(19) Europäisches Patentamt
European Patent Office
Office européen des brevets

(11) EP 1 204 660 B1

(12) EUROPEAN PATENT SPECIFICATION

(45) Date of publication and mention of the grant of the patent:
**20.10.2004 Bulletin 2004/43**

(21) Application number: **00950264.2**

(22) Date of filing: **21.07.2000**

(51) Int Cl.[7]: **C07D 409/04**, A61K 31/4535, A61P 25/24, A61P 3/04

(86) International application number:
**PCT/US2000/017864**

(87) International publication number:
**WO 2001/009126 (08.02.2001 Gazette 2001/06)**

(54) **SEROTONERGIC BENZOTHIOPHENES**

SEROTONERGE BENZOTHIOPHENE

BENZOTHIOPHENES SEROTONINERGIQUES

(84) Designated Contracting States:
**AT BE CH CY DE DK ES FI FR GB GR IE IT LI LU MC NL PT SE**

(30) Priority: **29.07.1999 US 146185 P**
**20.12.1999 US 172784 P**

(43) Date of publication of application:
**15.05.2002 Bulletin 2002/20**

(73) Proprietor: **ELI LILLY AND COMPANY**
**Indianapolis, Indiana 46285 (US)**

(72) Inventors:
- **BRINER, Karin**
  **Indianapolis, IN 46256 (US)**
- **BURKHOLDER, Timothy, Paul**
  **Carmel, IN 46032 (US)**
- **CONWAY, Richard, Gerard**
  **Carmel, IN 46032 (US)**
- **CUNNINGHAM, Brian, Eugene**
  **Indianapolis, IN 46237 (US)**
- **FINLEY, Don, Richard**
  **Greenwood, IN 46142 (US)**
- **HEINZ, Lawrence, Joseph**
  **Pittsboro, IN 46167 (US)**
- **JESUDASON, Cynthia, Darshini**
  **Indianapolis, IN 46228 (US)**
- **KOHLMAN, Daniel, Timothy**
  **Camby, IN 46113 (US)**
- **LIANG, Sidney, Xi**
  **Fishers, IN 46038 (US)**
- **XU, Yao-Chang**
  **Fishers, IN 46038 (US)**

(74) Representative: **Hiscock, Ian James et al**
**European Patent Operations,**
**Eli Lilly and Company Limited,**
**Lilly Research Centre,**
**Erl Wood Manor,**
**Sunninghill Road**
**Windlesham GU20 6PH (GB)**

(56) References cited:

| | |
|---|---|
| **EP-A- 0 398 413** | **EP-A- 0 535 722** |
| **EP-A- 0 982 304** | **WO-A-00/00196** |
| **WO-A-00/00303** | **US-A- 5 436 246** |

- **PATENT ABSTRACTS OF JAPAN vol. 016, no. 071 (C-0913), 21 February 1992 (1992-02-21) & JP 03 264583 A (DAI ICHI SEIYAKU CO LTD), 25 November 1991 (1991-11-25)**

EP 1 204 660 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The neurotransmitter serotonin (5-hydroxytryptamine, 5-HT) has a rich pharmacology arising from a heterogeneous population of at least seven receptor classes. The serotonin 5-$HT_2$ class is further subdivided into at least three subtypes, designated 5-$HT_{2a}$, 5-$HT_{2b}$, and 5-$HT_{2c}$. The 5-$HT_{2c}$ receptor has been isolated and characterized (Julius, *et al*., U.S. Patent No. 4,985,352), and transgenic mice lacking the 5-$HT_{2c}$ receptor have been reported to exhibit seizures and an eating disorder resulting in increased consumption of food (Julius, *et al*., U.S. Patent No. 5,698,766). Compounds selective for the 5-$HT_{2c}$ receptor would provide useful therapies for the treatment of seizure and eating disorders without the side effects associated with current therapies. The present invention provides new benzothiophenes that are useful 5-$HT_{2c}$ receptor agonists.

The present invention provides benzothiophenes of Formula I:

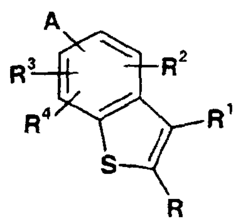

I

where:

R is hydrogen, halo, trifluoromethyl or $C_1$-$C_6$ alkyl;
$R^1$ is hydrogen, halo, trifluoromethyl, phenyl, or $C_1$-$C_6$ alkyl;
$R^2$, $R^3$, and $R^4$ are independently hydrogen, halo, trifluoromethyl, cyano, $C_1$-$C_4$ alkoxy, $C_1$-$C_4$ alkoxycarbonyl, $C_1$-$C_6$ alkyl, $C_1$-$C_6$ alkyl substituted with a substituent selected from the group consisting of $C_1$-$C_4$ alkoxy and hydroxy, or -C(O)NH$R^9$;
$R^9$ is $C_1$-$C_8$ alkyl where the alkyl chain is optionally substituted with a substituent selected from the group consisting of phenyl and pyridyl;
A is attached at either the 4- or 7-position of the benzothiophene nucleus and is an amine of formula:

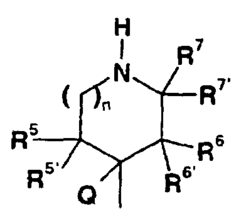

(i)

n is 0, 1, or 2;
$R^5$, $R^6$, and $R^7$ are independently hydrogen or $C_1$-$C_4$ alkyl;
Q is hydrogen;
$R^{5'}$ is hydrogen or methyl, provided that $R^{5'}$ may be methyl only when $R^5$ is other than hydrogen, or $R^{5'}$ and Q taken together with the carbon atoms to which they are attached form a double bond;
$R^{6'}$ is hydrogen or methyl, provided that $R^{6'}$ may be methyl only when $R^6$ is other than hydrogen, or $R^{6'}$ and Q taken together with the carbon atoms to which they are attached form a double bond;
$R^{7'}$ is hydrogen or methyl, provided that $R^{7'}$ may be methyl only when $R^7$ is other than hydrogen;

or pharmaceutically acceptable acid addition salts thereof subject to the following provisos:

a) when n is 1 or 2, at least one of $R^5$, $R^6$, and $R^7$, must be other than hydrogen; and

b) no more than two of $R^5$, $R^{5'}$, $R^6$, $R^{6'}$, $R^7$, and $R^{7'}$ may be other than hydrogen.

This invention also provides a pharmaceutical formulation which comprises, in association with a pharmaceutically acceptable carrier, diluent or excipient, a compound of Formula I.

The present invention provides a method for increasing activation of the 5-$HT_{2C}$ receptor in mammals comprising administering to a mammal in need of such activation a pharmaceutically effective amount of a compound of Formula I

The present invention also provides a method for treating obesity in mammals comprising administering to a mammal in need of such treatment a pharmaceutically effective amount of a compound of Formula I.

A further embodiment of this invention is a method for increasing activation of the 5-$HT_{2C}$ receptor for treating a variety of disorders that have been linked to decreased neurotransmission of serotonin in mammals. These disorders include depression, obesity, bulimia, premenstrual syndrome or late luteal phase syndrome, alcoholism, tobacco abuse, panic disorder, anxiety, post-traumatic syndrome, memory loss, dementia of aging, social phobia, attention deficit hyperactivity disorder, disruptive behavior disorders, impulse control disorders, borderline personality disorder, obsessive compulsive disorder, chronic fatigue syndrome, premature ejaculation, erectile difficulty, anorexia nervosa, disorders of sleep, autism, anxiety, seizure disorders, and mutism. Any of these methods employ a compound of Formula I.

This invention also provides the use of a compound of Formula I for the manufacture of a medicament for the treatment of obesity. Additionally, this invention provides a pharmaceutical formulation adapted for the treatment of obesity containing a compound of Formula I. Furthermore, this invention includes a method for the treatment of obesity which comprises administering an effective amount of a compound of Formula I.

The general chemical terms used in the formulae above have their usual meanings. For example, the term "alkyl" includes such groups as methyl, ethyl, n-propyl, isopropyl, n-butyl, isobutyl, sec-butyl, tert-butyl, and the like. The term "alkoxy" includes methoxy, ethoxy, propoxy, isopropoxy, butoxy and the like. The term "acyl" includes such groups as formyl, acetyl, propionyl, butyryl, 2-methylpropionyl, and the like. The term "halo" includes fluoro, chloro, bromo and iodo.

The term "$C_1$-$C_6$ alkyl substituted with a substituent selected from the group consisting of $C_1$-$C_4$ alkoxy and hydroxy" means a branched or linear alkyl group substituted in the carbon chain with one or two substituents independently selected from hydroxy or $C_1$-$C_4$ alkoxy.

The term "$C_1$-$C_8$ alkyl where the alkyl chain is optionally substituted with a substituent selected from the group consisting of phenyl and pyridyl" means a branched or linear alkyl group which may be substituted in the carbon chain with a phenyl or pyridinyl ring.

Since the compounds of this invention are amines, they are basic in nature and accordingly react with any of a number of inorganic and organic acids to form pharmaceutically acceptable acid addition salts. Since some of the free amines of the compounds of this invention are typically oils at room temperature, it is preferable to convert the free amines to their pharmaceutically acceptable acid addition salts for ease of handling and administration, since the latter are routinely solid at room temperature. Acids commonly employed to form such salts are inorganic acids such as hydrochloric acid, hydrobromic acid, hydroiodic acid, sulfuric acid, phosphoric acid, and the like, and organic acids, such as p-toluenesulfonic acid, methanesulfonic acid, oxalic acid, p-bromophenylsulfonic acid, carbonic acid, succinic acid, citric acid, benzoic acid, acetic acid and the like. Examples of such pharmaceutically acceptable salts thus are the sulfate, pyrosulfate, bisulfate, sulfite, bisulfite, phosphate, monohydrogen-phosphate, dihydrogenphosphate, metaphosphate, pyro-phosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, β-hydroxybutyrate, glycollate, tartrate, methanesulfonate, propanesulfonate, naphthalene-1-sulfonate, naphthalene-2-sulfonate, mandelate and the like. Preferred pharmaceutically acceptable salts are those formed with hydrochloric acid and fumaric acid.

The skilled artisan will appreciate that substituents on moiety "A" of certain compounds of the present invention give rise to cis- and trans-isomers. An example of this isomeric relationship is illustrated by the following benzothienylpiperidines:

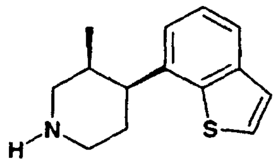

cis-isomer

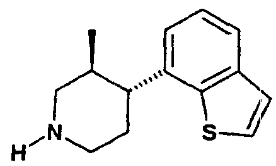

trans-isomer

The compounds of the invention may exist as a mixture of cis- and trans-isomers or as the individual isomers. It is preferred that the compounds exist as the individual isomers. Compounds in the cis- configuration are especially preferred.

The skilled artisan will also appreciate that the compounds of the present invention have at least one chiral carbon, and may therefore exist as a racemate, as individual enantiomers or diastereomers, and as mixtures of individual enantiomers or diastereomers. Individual enantiomers of compounds of the invention are illustrated by the following structures where $R^6$ or $R^7$ are other than hydrogen:

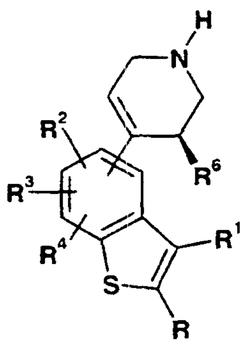

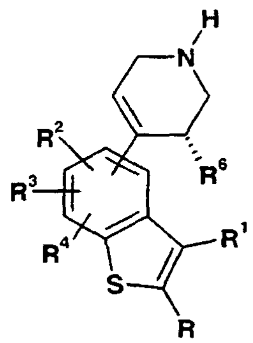

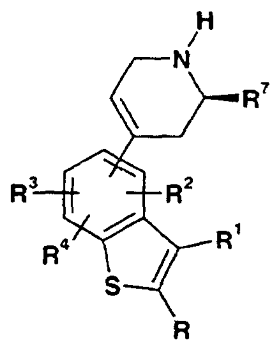

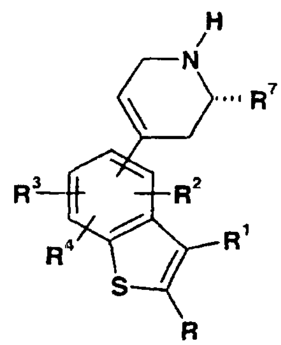

Individual diastereomers are illustrated by the following structures:

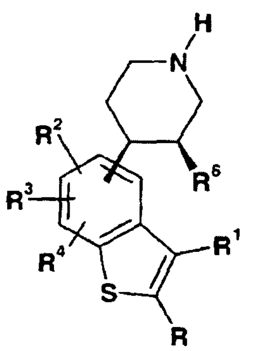

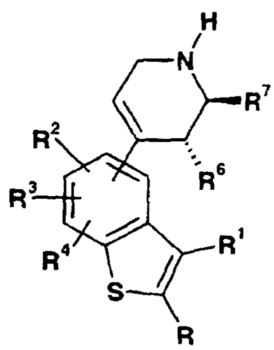

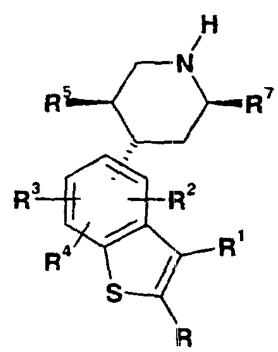

The enantiomers and diastereomers illustrated above are representative of other enantiomers and diasteromers created by other combinations of certain non-hydrogen substituents on the compounds of the invention, and are not intended to limit the scope of the present invention in any way. Furthermore, the skilled artisan will appreciate that certain substituents on the benzothienyl ring of the compounds of the invention introduce additional asymmetric centers into the molecule, creating additional optical isomers as described above.

While it is a preferred embodiment of the invention that the compounds of the invention exist, are formulated, and are used as single enantiomers or diastereomers, the present invention also contemplates the compounds of the invention existing in racemic form and as mixtures of the individual enantiomers and diastereomers. The methods and formulations of the invention also contemplate the use and formulation of the compounds of the invention in their

racemic form and as mixtures of the individual enantiomers and diastereomers.

The individual enantiomers and diastereomers may be prepared by chiral chromatography of the racemic or enantiomerically or diastereomerically enriched free amine, or fractional crystallization of salts prepared from racemic or enantiomerically or diastereomerically enriched free amine and a chiral acid. Alternatively, the free amine may be reacted with a chiral auxiliary and the enantiomers or diastereomers separated by chromatography followed by removal of the chiral auxiliary to regenerate the free amine. Furthermore, separation of enantiomers or diastereomers may be performed at any convenient point in the synthesis of the compounds of the invention. The compounds of the invention may also be prepared by the use of chiral syntheses. A particularly useful method for the separation of enantiomers or diastereomers is the "Dutch Resolution" described in EP 0 838 448 A1 (See also: T. Vries, et al., Angew. Chem. Int. Ed., **37**, 2349-2354 (1998); and B. Broxterman, et al., Chim. Oggi., **16**, 34-37 (1998)). Especially useful salts for resolution include 3-bromocamphor-8-sulfonic acid, mandelic acid, dibenzoyltartaric acid, di-(p-methylbenzoyl)tartaric acid, and di-(p-methoxybenzoyl)tartaric acid.

While all of the compounds of Formula I are useful 5-$HT_{2c}$ agonists, certain classes of the compounds are preferred. The following paragraphs describe such preferred classes:

aa) A is attached at the 7-postion of the benzothiophene ring;
ab) n is 0 or 1;
ac) n is 1;
ad) Q is hydrogen;
ae) Q and $R^{5'}$, taken together with the carbon atoms to which they are attached, form a double bond;
af) Q and $R^{6'}$, taken together with the carbon atoms to which they are attached, form a double bond;
ag) R is hydrogen;
ah) R is halo;
ai) R is $C_1$-$C_6$ alkyl;
aj) $R^1$ is hydrogen;
ak) $R^1$ is halo;
al) $R^1$ is trifluoromethyl;
am) $R^1$ is $C_1$-$C_6$ alkyl;
an) $R^2$ is hydrogen;
ao) $R^2$ is halo;
ap) $R^2$ is fluoro;
aq) $R^2$ is trifluoromethyl;
ar) $R^3$ is hydrogen;
as) $R^3$ is halo;
at) $R^3$ is fluoro;
au) $R^3$ is trifluoromethyl;
av) $R^4$ is hydrogen;
aw) $R^4$ is halo;
ax) $R^4$ is fluoro;
ay) $R^4$ is trifluoromethyl;
az) $R^5$ is hydrogen;
ba) $R^5$ is $C_1$-$C_4$ alkyl;
bb) $R^5$ is methyl;
bc) $R^{5'}$ is hydrogen;
bd) $R^{5'}$ is methyl;
be) $R^6$ is hydrogen;
bf) $R^6$ is $C_1$-$C_4$ alkyl;
bg) $R^6$ is methyl;
bh) $R^{6'}$ is hydrogen;
bi) $R^{6'}$ is methyl;
bj) $R^7$ is hydrogen;
bk) $R^7$ is $C_1$-$C_4$ alkyl;
bl) $R^7$ is methyl;
bm) $R^{7'}$ is hydrogen;
bn) $R^{7'}$ is methyl;
bo) The compound is a free base;
bp) The compound is a salt;
bq) The compound is the hydrochloride salt;

br) The compound is the fumarate salt;
bs) The compound is a racemate;
bt) The compound is a single enantiomer;
bu) The compound is a single diastereomer;
bv) A is attached at the 7-position of the benzothienyl moiety and only one of $R^2$, $R^3$, or $R^4$ is hydrogen;
bw) A is attached at the 7-position of the benzothienyl moiety and any two of $R^2$, $R^3$, or $R^4$ is hydrogen;
bx) A is attached at the 7-position of the benzothienyl moiety and all three of $R^2$, $R^3$, and $R^4$ are other than hydrogen;
by) One of $R^5$, $R^6$, and $R^7$ is other than hydrogen;
bz) Two of $R^5$, $R^{5'}$, $R^6$, $R^{6'}$, $R^7$, and $R^{7'}$ are other than hydrogen;
ca) n is 1 and A is monosubstituted in the three position;
cd) n is 1 and A is substituted in both the two and three positions.

It will be understood that the above classes may be combined to form additional preferred classes.

The present invention also provides a method for increasing activation of the 5-$HT_{2C}$ receptor in mammals by administering to a mammal in need of such activation a pharmaceutically effective amount of a compound of Formula I. The preferred mammal is human.

The compounds of the invention may be prepared beginning with an appropriately substituted benzothiophene and a suitable ketone as illustrated in the following scheme, where X is bromo or iodo, Pg is a nitrogen protecting group; and the variables n, R, $R^1$, $R^2$, $R^3$, $R^4$, $R^5$, $R^{5'}$, $R^6$, $R^{6'}$, $R^7$, and $R^{7'}$ are as previously defined:

Synthetic Scheme I

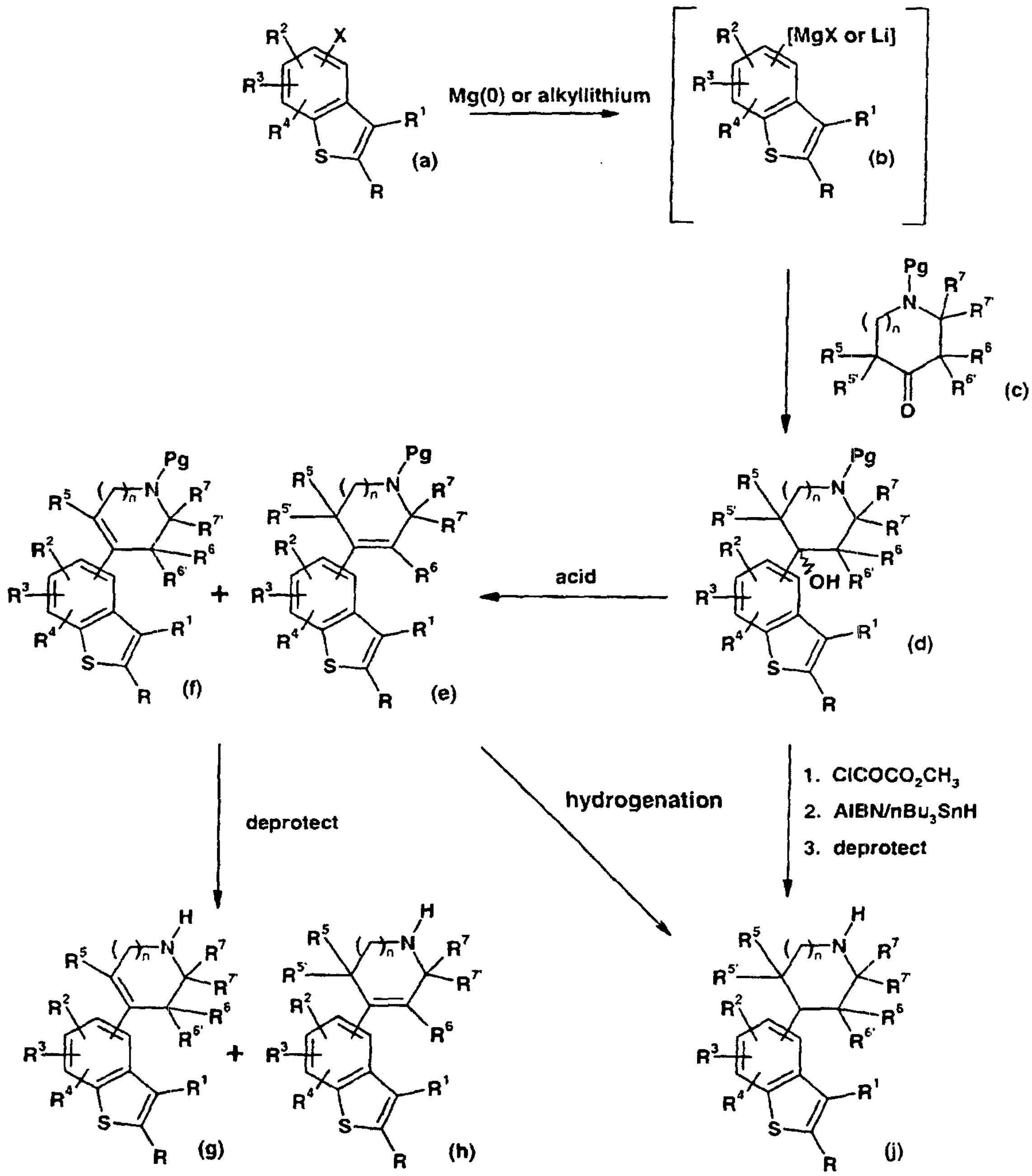


An appropriately substituted bromo- or iodobenzothiophene (a) in an appropriate solvent, typically tetrahydrofuran or diethyl ether, is treated with magnesium metal to prepare the corresponding Grignard reagent (b). Alternatively, a solution of the bromo- or iodobenzothiophene in a suitable solvent, typically diethyl ether or tetrahydrofuran, is treated with an alkyllithium, typically tert-butyllithium, to prepare the corresponding organolithium reagent (b). Either of these reagents is then reacted with an appropriately substituted, nitrogen protected, pyrrolidinone (n=0), piperidinone (n=1), or homopiperidinone (n=2) (c) to prepare the benzyl alcohol (d). Nitrogen protecting groups useful for this reaction are well known to the skilled artisan. A summary of such groups may be found in Greene's Protective Groups in Organic Synthesis, Second Edition, Wiley Interscience. Particularly useful protecting groups include benzyl and tert-butoxycarbonyl.

The skilled artisan will appreciate that benzothiophenes where R is hydrogen may be too acidic to tolerate the reaction conditions previously described. The 2-position of these benzothiphenes may be protected by treatment with an alkyllithium, typically n-butyllithium, at low temperature and then quenching the resulting anion solution with trimethylsilyl chloride. The resulting 2-trimethylsilylbenzothiophene may be deprotected by treatment with acid at any convenient point in the synthesis of the compounds of the present invention.

The benzyl alcohol (d) is dehydrated by treatment with an acid, typically p-toluenesulfonic acid, methanesulfonic acid, hydrochloric acid or hydrobromic acid, to provide a mixture of alkenes. When Pg is a moiety stable to acidic dehydration conditions, exemplified by compounds where Pg is benzyl, the alkenes of formula (e) and (f) are prepared. When Pg is labile to acidic conditions, exemplified by compounds where Pg is tert-butoxycarbonyl, the compounds of formula (g) and (h) are prepared directly. The alkenes may be conveniently hydrogenated over a catalyst, preferably a palladium catalyst, to provide the corresponding piperidines. Either of these mixtures of alkenes may be separated into their individual isomers by chromatography or fractional crystallization. The compounds may then be nitrogen deprotected, if necessary, to provide compounds of the present invention.

An alternative to the acidic dehydration of the benzyl alcohol (d) followed by hydrogenation sequence is a tin mediated deoxygenation. This deoxygenation is accomplished by first converting the benzylic alcohol to the corresponding oxalate ester by treatment with methyl chlorooxalate under standard acylation conditions. The resulting derivative is treated with tri(n-butyl)tin hydride in the presence of 2,2'-azobisisobutyronitrile to provide the compound of formula (j). This compound is then nitrogen deprotected to provide compounds of the present invention.

An alternative to the anion based coupling described above is the palladium catalyzed coupling described in the following scheme, where the variables are as previously described.

Synthetic Scheme II

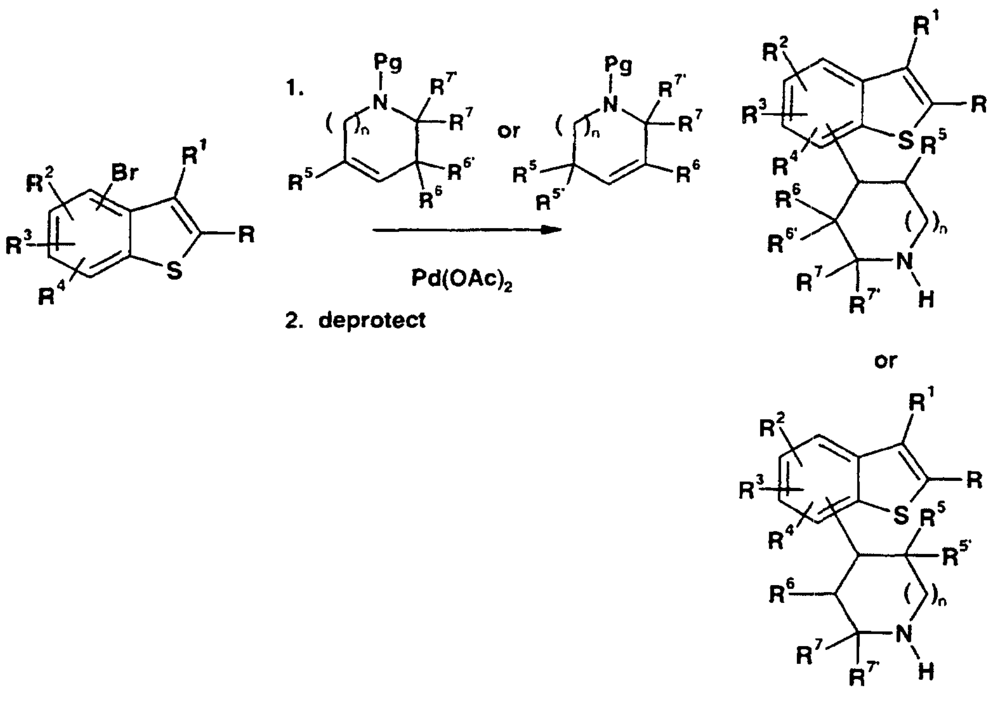


An appropriately substituted bromobenzothiophene and the requisite N- protected dihydropyrrole (n=0), tetrahydropyridine (n=1) or didehydrohomopiperidine (n=2) in an appropriate solvent, typically dimethylformamide, is heated with a source of palladium, such as palladium acetate, an appropriate phosphine, such as tri(fur-2-yl)phosphine, an appropriate amine, such as diisopropylethylamine, and lithium chloride. The product of this coupling reaction is then nitrogen deprotected to provide compounds of the present invention.

Alternatively, the palladium-mediated coupling may be performed on an appropriately substituted benzothienylboronic acid derivative and an appropriate enoltriflate derivative of the corresponding pyrrolidinone, piperidinone, or homopiperidinone. These coupling partners may be prepared from the corresponding bromobenzothiophenes and ketones respectively by procedures well known in the art. This type of palladium coupling reaction is well known in the

art (See: N. Miyaura et al., Journal of Organic Chemistry, **51**, 5467-5471 (1986); Y. Hoshino, et al., Bull. Chim. Soc. Jap., **61**, 3008-3010 (1988); N. Miyaura et al., Journal of the American Chemical Society, **111**, 314-321 (1989); W. J. Thompson, et al., Journal of Organic Chemistry, **53**, 2052-2055 (1988); and T. I. Wallow and B. M. Novack, Journal of Organic Chemistry, **59**, 5034-5037 (1994)).

The skilled artisan will appreciate that the conditions for any of the nitrogen deprotection steps described above depend upon the nature of the nitrogen protecting group. For example, the benzyl group may be removed by treatment with 1-chloroethyl chloroformate or by catalytic hydrogenation. The tert-butoxycarbonyl group may be removed by treatment with acid, for example, trifluoroacetic acid or hydrogen chloride.

The benzothiophenes required for the preparation of the novel compounds useful for the method of the present invention are either commercially available or may be prepared by methods well known to the skilled artisan. For example, Method (a) of Synthetic Scheme III is that of Beck et al. (Journal of Organic Chemistry, **37**(21), 3224 (1972)); and Method (b) of is that of Bridges et al., Tetrahedron Letters, **33**(49), 7499 (1992). $R^2$, $R^3$ and $R^4$ are as previously defined.

Synthetic Scheme III

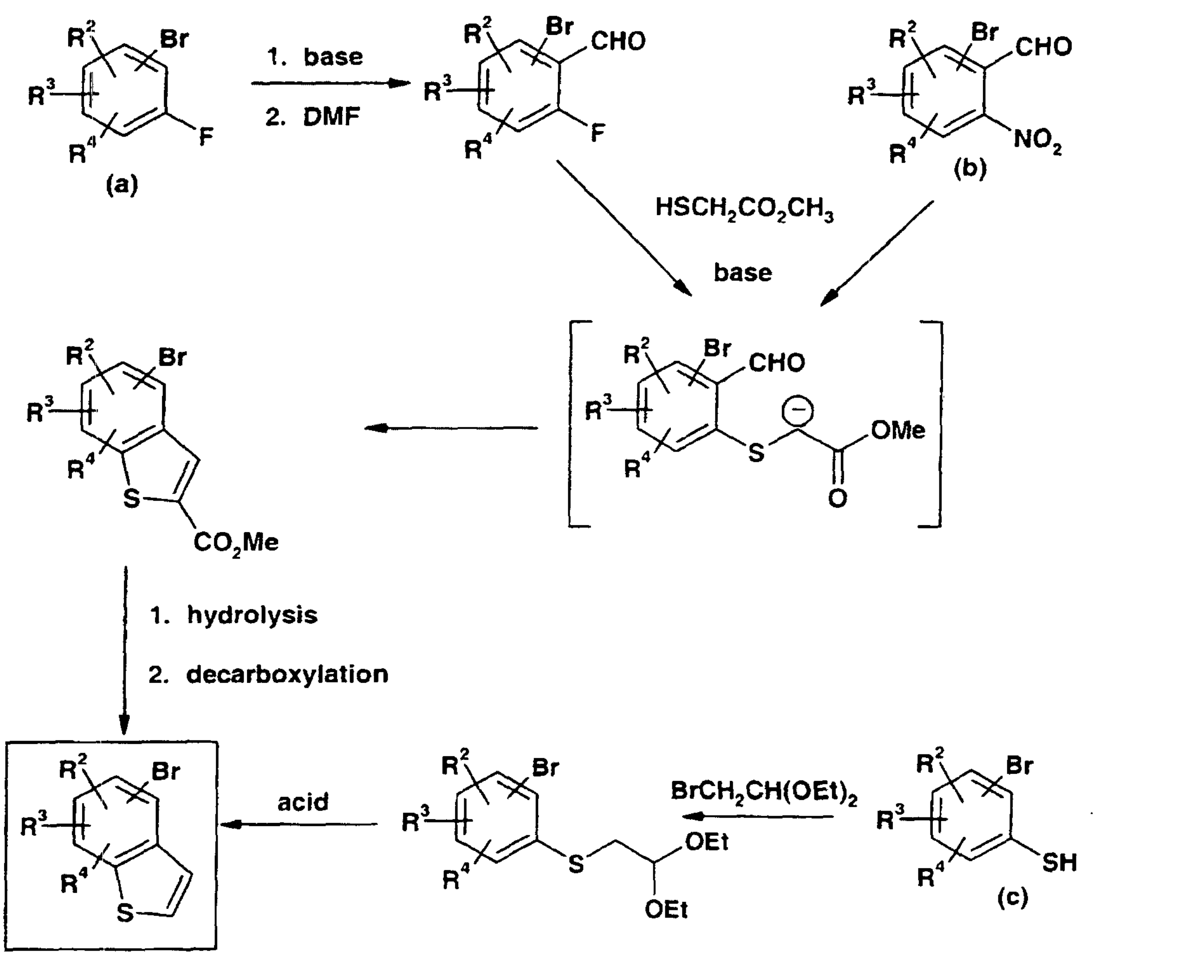


The three methods described in Synthetic Scheme IV provide the requisite benzothiophenes from three different structural classes of starting materials. The selection of a particular method is dependent upon the availability of starting materials and the stability of the substituents to the particular reaction conditions.

Method (a) of Synthetic Scheme III takes advantage of the relative acidity of aromatic protons adjacent to a carbon bearing a fluorine atom. Treatment of an appropriate fluorobenzene with a suitable base followed by addition of dimethylformamide provides, after an aqueous acid workup, the corresponding fluorobenzaldehyde. Suitable bases for this transformation include alkyllithiums such as n-butyllithium or sec-butyllithium, and lithium amides such as lithium 2,2,6,6-tetramethylpiperidide or lithium diisopropylamide. The resulting fluorobenzaldehyde is treated with the anion

of methyl thioglycollate. This anion may first be formed by treatment of a solution of methyl thioglycollate in dimethylsulfoxide with a metal hydride, preferably sodium hydride, and then adding the fluorobenzaldehyde. The exothermic reaction provides the corresponding methyl benzothiophene-2-carboxylate. Alternatively, the fluorobenzaldehyde, methyl thioglycollate and a suitable tertiary amine, preferably triethylamine, are heated together in dimethylsulfoxide to prepare the corresponding methyl benzothiophene-2-carboxylate.

An alternate route to the same methyl benzothiophene-2-carboxylate is illustrated by method (b) of Synthetic Scheme III. This method exploits the facility with which an aromatic nitro group can undergo nucleophilic displacement. A suitable o-nitrobenzaldehyde is treated with an equimolar amount of methyl thioglycollate and potassium carbonate in dimethylformamide.

The methyl benzothiophene-2-carboxylates prepared by either of these two methods is converted to the required benzothiophene by standard ester hydrolysis/decarboxylation steps. A solution of the appropriate ester in a lower alkanol, typically methanol or ethanol, is treated with a small excess of sodium or potassium hydroxide. Once the hydrolysis is complete, volatiles are removed under reduced pressure. The residue is taken up in quinoline and to this mixture is added elemental copper. The reaction mixture is then heated to about 200°C until the decarboxylation is complete. The desired product is isolated by normal extractive techniques and may be purified by chromatography or crystallization as appropriate prior to subsequent use.

Method (c) provides the requisite benzothiophenes from appropriately substituted thiophenols, including aminothiophenols. A solution of the thiophenol in an appropriate solvent, such as acetone, tetrahydrofuran or diethyl ether, is treated with potassium carbonate followed by bromoacetaldehyde diethyl acetal. The resulting mixture is stirred at about ambient temperature for from 1 hour to about 48 hours until the reaction is complete. The reaction mixture is then filtered and the filtrate concentrated under reduced pressure. The residue is subjected to an extractive workup and the product may be used directly in the subsequent step or purified by chromatography or crystallization if desired. This material is then dissolved in an appropriate solvent, typically a halobenzene such as chlorobenzene, and is treated with a suitable acid, typically polyphosphoric acid. The reaction is heated to reflux until the cyclization is complete. The desired benzothiophene may be isolated by normal extractive workups. In those cases where substituents are such that isomeric benzothiophenes may result from the cyclization, the isomers may be separated by chromatographic or crystallization techniques at this or any subsequent convenient point in the synthetic pathway to compounds of the present invention.

The skilled artisan will appreciate that the requisite thiophenols are either commercially available or may be prepared by methods well known to those skilled in the art.

Those compounds of the present invention where the benzothiophene ring is substituted with hydroxy are easily prepared by trimethylsilyl iodide cleavage of the corresponding alkoxy compound, or catalytic O-debenzylation of the corresponding benzyloxy compound. This hydrogenolysis may be performed by dissolution of an appropriate substrate in a lower alkanol, such as methanol or ethanol, tetrahydrofuran or a mixed solvent system of tetrahydrofuran and ethyl acetate. The hydrogenation may be performed at an initial hydrogen pressure of 20-80 p.s.i., preferably from 50-60 p.s.i., at 0-60°C, preferably at ambient temperature to 40°C, for 1 hour to 3 days. Additional charges of hydrogen may be required to drive the reaction to completion depending on the specific substrate. Compounds prepared in this manner are isolated by removal of the catalyst by filtration followed by concentration of the reaction solvent under reduced pressure. The recovered product may be purified by chromatography or recrystallization from a suitable solvent if necessary.

The skilled artisan will appreciate that benzothiophenes substituted in the 2- and/or 3-position may be prepared by modification of the chemistry described in Synthetic Scheme III. For example, a thiophenol may be alkylated with a suitable haloketone and then cyclized to provide a substituted benzothiophene. Alternatively, the benzothienyl moiety may be substituted in the 2- or 3-position at any convenient point in the synthesis of the compounds of the present invention by methods known to those skilled in the art.

The requisite pyrrolidinones (n=0) and piperidinones (n=1) are either commercially available or may be prepared by methods well known in the art. The requisite homopiperidinones (n=2) may be prepared from appropriately substituted piperidinones. One such approach is a modification of a synthesis described by Roglans, et al., Synthetic Communications, **22**(9), 1249-1258 (1992). Procedures for the preparation of pyrrolidinones and homopiperidinones are described in the following scheme where the variables are as previously described.

Synthetic Scheme IV

where n = 0

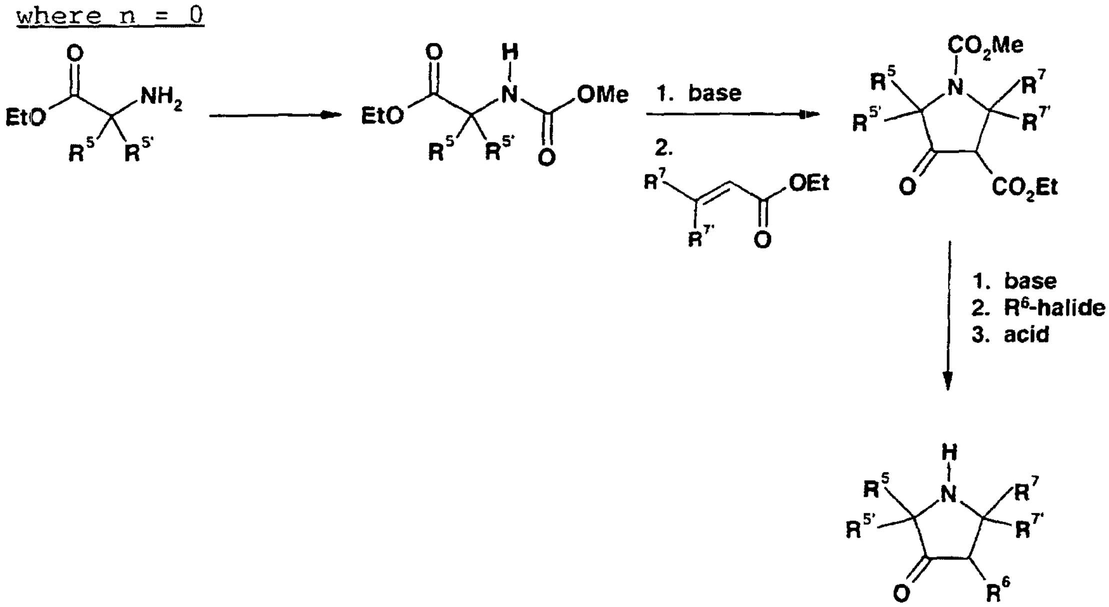


where n = 2

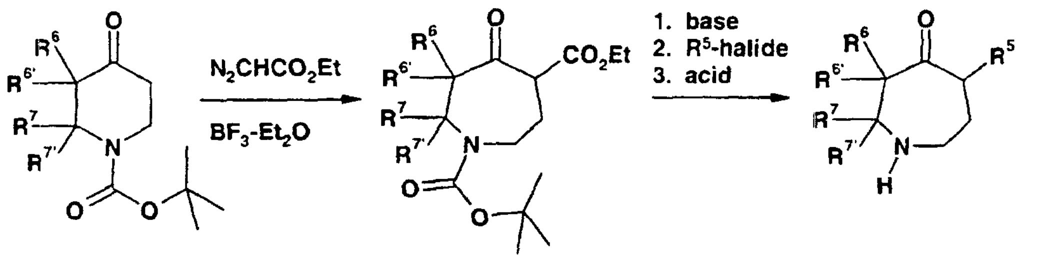


The requisite pyrrolidinones (n = 0) may be prepared by reacting an ester, for example the ethyl ester, of an •-amino acid with methyl chloroformate. The resulting carbamate is treated with base and then with an appropriately substituted acrylic acid ester. The resulting •-keto ester is treated with base and an appropriate alkylating agent, such as an alkyl halide, followed by decarboxylation and deprotection in the presence of aqueous acid to provide the desired compound.

The requisite homopiperidinones may be prepared from the corresponding piperidine via standard ring expansion protocols employing ethyl diazoacetate and an appropriate Lewis acid, such as boron trifluoride. The resulting •-keto ester is treated with base and an appropriate alkylating agent, such as an alkyl halide, followed by decarboxylation and deprotection in the presence of aqueous acid to provide the desired compound. The skilled artisan will appreciate that subsequent transformations are possible, if necessary or desired, to prepare more highly substituted compounds.

The skilled artisan will appreciate that not all substituents are compatible with the reaction conditions employed to prepare the compounds of the invention. Those substituents incompatible with these conditions may be introduced at a more convenient point in the synthesis, or may be prepared by functional group transformations well known to one of ordinary skill in the art. Furthermore, many of the compounds of the present invention, while useful 5-$HT_{2C}$ agonists in their own right, are useful intermediates to prepare other compounds of the invention. Those compounds of the invention bearing an ester functionality, for example, may be hydrolyzed under standard conditions to provide the corresponding carboxylic acids. These acids may then be reacted with amines under standard peptide coupling conditions to provide the corresponding amides. Alternatively, the esters may be reduced to provide the corresponding alcohols. Furthermore, alkoxy groups may be cleaved to provide the corresponding phenols, and primary amines may be diazotized and displaced to provide the corresponding halogenated compounds.

The following Preparations and Examples are illustrative of methods useful for the synthesis of the compounds of the present invention.

The 2-trimethylsilylbenzothiophenes necessary for the preparation of the compounds of the present invention may be prepared essentially as described in Preparation I.

Preparation I

2-trimethylsilyl-6-fluorobenzothiophene

A solution of 13.6 gm (89.2 mMol) 6-fluorobenzothiophene in 59 mL tetrahydrofuran was cooled to -78°C. To this solution were added 66.9 mL (107.1 mMol) n-butyllithium (1.6 M in hexane) at a rate to maintain the reaction mixture at or below -65°C. The reaction mixture was stirred for one hour while allowing to warm to -60°C. At this point 13.6 mL (107.1 mMol) trimethylsilyl chloride were added over 10 minutes. The reaction mixture was allowed to warm to room temperature. After about 1 hour, the reaction mixture was quenched by the addition of 75 mL saturated aqueous ammonium chloride and 25 mL water. The resulting mixture was diluted with 100 mL hexane and the phases separated. The aqueous phase was extracted 2 x 100 mL hexane. The organic phases were combined, washed with saturated aqueous sodium chloride, dried over sodium sulfate and concentrated under reduced pressure to provide 19.4 gm (97%) of the title compound as a crystalline solid.
m.p. = 40-42°C

The thiophenols necessary for the preparation of the compounds of the present invention may be prepared from the corresponding phenols essentially by the procedure described in Preparation II.

Preparation II

2-bromo-5-fluorothiophenol

A solution of 12.9 gm (105 mMol) N,N-dimethylthiocarbamoyl chloride in dimethylformamide was added to a mixture of 10 gm (52 mMol) 2-bromo-5-fluorophenol, and 11.7 gm (105 mMol) 1,4-diazabicyclo[2,2,2]octane in 100 mL dimethylformamide. The reaction mixture was stirred at room temperature for 2 hours. The mixture was then diluted with water and the aqueous phase washed well with ethyl acetate. The combined organic phases were washed sequentially with 1N hydrochloric acid, water, and saturated aqueous sodium chloride, dried over sodium sulfate and concentrated under reduced pressure.

This residue was dissolved in diphenyl ether and heated at 230-240°C for about 4 hours. The reaction mixture was cooled to room temperature and was then poured over a silica gel column. The column was eluted first with hexane to remove the diphenyl ether, and then with a gradient of hexane containing 5-20% ethyl acetate. Fractions containing the desired compound were combined and concentrated under reduced pressure to provide 11 gm of the desired compound.

A mixture of 2.5 gm (9.0 mMol) of the resulting carbamate and 1.5 gm (27 mMol) potassium hydroxide in 50 mL methanol was heated at reflux for 1.5 hours. The reaction mixture was then diluted with water and extracted well with diethyl ether. The aqueous phase was then made acidic with hydrochloric acid and extracted well with ethyl acetate. These organic phases were combined, washed with saturated aqueous sodium chloride, dried over sodium sulfate and concentrated under reduced pressure. The residue was purified by silica gel chromatography to provide 1.7 gm (91%) of the title compound.

Preparation III

4-fluoro-7-bromobenzothiophene

2-(2-bromo-5-fluorophenylthio)acetaldehyde diethylacetal

A mixture of 1.9 gm (9.66 mMol) 2-bromoacetaldehyde diethylacetal and 2.6 gm (19.3 mMol) potassium carbonate in 100 mL dimethylformamide was deoxygenated by bubbling in nitrogen for 30 minutes. To this mixture were added 2.0 gm (9.66 mMol) 2-bromo-5-fluorothiophenol and the reaction mixture was stirred at room temperature for about 64 hours. The reaction mixture was then diluted with water and extracted well with ethyl acetate. The organic extracts were combined, washed well with water, dried over sodium sulfate, and concentrated under reduced pressure to provide 2.95 gm (95%) of the desired compound.

Cyclization

A mixture of 20 gm polyphosphoric acid in 300 mL chlorobenzene was heated at reflux. To this mixture was

added a solution of 5 gm 2-(2-bromo-5-fluorophenylthio)acetaldehyde diethylacetal in 100 mL chlorobenzene dropwise. The reaction mixture was heated at reflux for about 16 hours. The reaction mixture was cooled to room temperature and the chlorobenzene decanted from the polyphosphoric acid. The chlorobenzene was concentrated under reduced pressure and the residue was dissolved in ethyl acetate. This solution was washed sequentially with 0.5 M sodium hydroxide and saturated aqueous sodium chloride, dried over sodium sulfate and concentrated under reduced pressure. The residue was subjected to silica gel chromatography, eluting with 5% ethyl acetate in hexane. Fractions containing product were combined and concentrated under reduced pressure to provide 2.4 gm (76%) of the title compound as a pale yellow solid.

Preparation IV

5-fluoro-7-bromobenzothiophene

A suspension of 10 gm AMBERLYST-15™ resin in 400 mL chlorobenzene was heated at reflux as most of the chlorobenzene was distilled away. The resin was dried and then resuspended in 400 mL chlorobenzene. This suspension was heated at reflux as a solution of 7 gm 2-(2-bromo-4-fluorophenylthio)acetaldehyde diethylacetal in 100 mL chlorobenzene was added dropwise over about 2 hours. Chlorobenzene was removed by distillation to maintain a constant volume of about 400 mL. When reaction was complete as determined by thin layer chromatography, the reaction mixture was cooled to room temperature and the resin removed by filtration. The resin was washed twice with dichloromethane and the combined filtrates were concentrated under reduced pressure. The residue was subjected to silica gel chromatography, eluting with 5% ethyl acetate in hexane. Fractions containing product were combined and concentrated under reduced pressure to provide 3 gm (75%) of the title compound as a light yellow oil which solidified upon standing at room temperature.

Preparation V

4-bromobenzothiophene and 6-bromobenzothiophene

Beginning with 36.3 gm (132 mMol) 2-(3-bromophenylthio)acetaldehyde ethylene glycol acetal, 15.6 gm (57%) of the title mixture were prepared essentially as described in Preparation III.

Preparation VI

1-benzyl-3-ethyl-4-oxopiperidine

Methyl 1-tert-butoxycarbonyl-4-oxo-3-piperidinecarboxylate

A mixture of 20 gm (103.3 mMol) methyl 4-oxo-3-piperidinecarboxylate hydrochloride and 75 mL saturated aqueous sodium bicarbonate in 150 mL dichloromethane was stirred vigorously at room temperature. A solution of 24.8 gm (113.6 mMol) di-tert-butyl dicarbonate in 100 mL dichloromethane was then added dropwise over three hours. After stirring at room temperature for about 16 hours, an additional 3.0 gm di-tert-butyl dicarbonate were added and stirring continued for an additional 2 hours. The reaction mixture was then diluted with water and extracted with 2 x 250 mL dichloromethane. The organic phases were combined, dried over magnesium sulfate, and concentrated under reduced pressure to provide 26.2 gm (99%) of the desired compound as a yellow oil.

Methyl 1-tert-butoxycarbonyl-4-oxo-3-ethyl-3-piperidinecarboxylate

A solution of 26 gm (101 mMol) methyl 1-tert-butoxycarbonyl-4-oxo-3-piperidinecarboxylate and 11.9 gm (106 mMol) potassium tert-butoxide in 300 mL tert-butanol was heated to 70°C under nitrogen. To this solution were then added 23.6 gm (152 mMol) iodoethane over 20 minutes, and the reaction mixture was heated at reflux for about 16 hours. The reaction mixture was then cooled to room temperature and was diluted with diethyl ether. The organics were washed with water, dried over magnesium sulfate, and concentrated under reduced pressure to provide 28.7 gm (99%) of the desired compound as a yellow oil.

3-ethyl-4-oxopiperidine hydrochloride

A mixture of 28.2 gm (98.8 mMol) methyl 1-tert-butoxycarbonyl-4-oxo-3-ethyl-3-piperidinecarboxylate and 6N hydrochloric acid was heated at reflux for about 16 hours. The reaction mixture was concentrated under reduced

pressure and the residue dissolved in ethanol. This solution was concentrated under reduced pressure to azeotropically remove water. This procedure was repeated twice to provide 7.0 gm (55%) of the desired compound.

Benzylation

A mixture of 7.0 gm (54.8 mMol ) 3-ethyl-4-oxopiperidine and 22.7 gm (164.4 mMol) potassium carbonate in 100 mL tetrahydrofuran was cooled to 0°C with vigorous stirring. A solution of 9.2 gm (53.7 mMol) benzyl bromide in 20 mL tetrahydrofuran was added dropwise over 20 minutes. The reaction mixture was then allowed to warm gradually to room temperature. After about 16 hours the reaction mixture was diluted with water and was extracted with 2 x 250 mL dichloromethane. The organic phases were combined, dried over magnesium sulfate and concentrated under reduced pressure. The residue was subjected to silica gel chromatography, eluting with a gradient of hexane containing from 5-15% ethyl acetate. Fractions containing product were combined and concentrated under reduced pressure to provide 9.4 gm (79%) of the title compound as a light yellow oil.

Preparation VII

1-benzyl-3,3-dimethyl-4-oxopiperidine

Methyl N-[benzyl] 3-aminopropionate

A solution of 55 mL (0.50 mole) benzylamine in 250 mL methanol was cooled in an ice bath. A solution of 54 mL (0.60 mole) methyl acrylate in 50 mL methanol was added dropwise over 30 minutes. The reaction mixture was then allowed to warm gradually to room temperature. After about 96 hours the reaction mixture was concentrated under reduced pressure and the residue vacuum distilled to provide 74.6 gm (77%) of the desired compound in two fractions.

Methyl N-[benzyl]-N-[methyl malonoyl] 3-aminopropionate

144 mL (1.25 mole) dimethyl malonate was heated at from 165 - 175°C. To this heated substrate was added 48.6 gm (0.25 mole) methyl N-[benzyl] 3-aminopropionate dropwise. Methanol was distilled off as the reaction proceeded. After 8.0 mL of methanol was recovered, the temperature of the reaction mixture was increased to 180 - 185°C. After 1 hour at this temperature, an additional 1.5 mL of methanol had been recovered. The reaction mixture was then cooled to room temperature and the excess dimethyl malonate was removed by vacuum distillation. The residue was subjected to silica gel chromatography, eluting with a gradient of hexane containing from 0-50% ethyl acetate. Fractions containing the desired product were combined and concentrated under reduced pressure to provide 55.7 gm (76%).

Methyl 1-benzyl-2,4-dioxo-3-piperidinecarboxylate

A mixture of 130 gm (0.94 mole) potassium carbonate and 5 gm (18.9 mMol) 18-crown-6 in 500 mL toluene was heated at reflux. A solution of 55 gm (188 mMol) methyl N-[benzyl]-N-[methyl malonoyl] 3-aminopropionate in 150 mL toluene was added dropwise over 30 minutes. After 6 hours the reaction mixture was cooled to room temperature and then diluted with 1L water. The toluene phase was diluted with 150 mL ethyl acetate and the phases separated. The organic phase was extracted with 2 x 250 mL water. The combined aqueous phases were cooled in an ice bath as 6N hydrochloric acid was added until pH<1. The aqueous phase was then extracted with 3 x 300 mL dichloromethane. The combined dichloromethane extracts were dried over magnesium sulfate and concentrated under reduced pressure to provide 23.4 gm (48%) of the desired compound. The toluene/ethyl acetate extract was washed with saturated aqueous sodium chloride and then concentrated under reduced pressure to provide 26.1 gm (47%) unreacted methyl N-[benzyl]-N-[methyl malonoyl] 3-aminopropionate.

1-benzyl-2,4-dioxopiperidine

A mixture of 23.4 gm (89.7 mMol) methyl 1-benzyl-2,4-dioxo-3-piperidinecarboxylate in 200 mL 10% aqueous oxalic acid was stirred at reflux for 15 hours. The reaction mixture was cooled to room temperature and the solution extracted with 3 x 200 mL dichloromethane. The organic phases were combined, dried over magnesium sulfate, and concentrated under reduced pressure to provide 4.99 gm (27%) of the desired compound.

1-benzyl-2,4-dioxo-3,3-dimethylpiperidine

A mixture of 1.44 gm (7.1 mMol) 1-benzyl-2,4-dioxopiperidine, 4.0 gm (28.9 mMol) potassium carbonate, and

1.5 mL (24.1 mMol) iodomethane in 14 mL dimethylsulfoxide was stirred for 24 hours. The reaction mixture was diluted with ethyl acetate and water. The phases were separated and the organic phases was washed sequentially with water and saturated aqueous sodium chloride, dried over magnesium sulfate and concentrated under reduced pressure. The residue was subjected to silica gel chromatography, eluting with hexane containing 35% ethyl acetate. Fractions containing product were combined and concentrated under reduced pressure to provide 0.61 gm (37%) of the desired compound.

1-benzyl-3,3-dimethyl-4-hydroxypiperidine

A solution of 0.30 gm (1.3 mMol) 1-benzyl-2,4-dioxo-3,3-dimethylpiperidine in 5 mL tetrahydrofuran was stirred at room temperature as 2 mL (2 mMol) lithium aluminum hydride (2M in tetrahydrofuran) was added dropwise. The reaction mixture was then heated to reflux for 3 hours. The reaction mixture was then allowed to cool to room temperature. After stirring at room temperature for about 16 hours, the reaction mixture was cooled in an ice bath and treated sequentially with 0.5 mL water, 0.5 mL 5N sodium hydroxide, and 0.5 mL water with vigorous stirring. The resulting slurry was diluted with dichloromethane and anhydrous sodium sulfate was added. The slurry was filtered and the filter cake washed with dichloromethane. The combined filtrates were concentrated under reduced pressure and the residue subjected to silica gel chromatography, eluting with dichloromethane containing from 0-10% methanol containing 0.1% ammonium hydroxide. Fractions containing product were combined and concentrated under reduced pressure to provide 0.25 gm (88%) of the desired compound.

Oxidation

A solution of 0.40 mL (5.6 mMol) dimethylsulfoxide in 5 mL dichloromethane was cooled to -78°C. To this solution was added 0.35 mL (2.48 mMol) trifluoroacetic anhydride dropwise and the resulting solution stirred for 30 minutes. A solution of 0.25 gm (1.14 mMol) 1-benzyl-3,3-dimethyl-4-hydroxypiperidine in 2 mL dichloromethane was added dropwise and the reaction mixture was stirred for 1 hour. To the solution was then added 1.0 mL (7.2 mMol) triethylamine and the reaction mixture was warmed to 0°C. After stirring for 2 hours, the reaction mixture was poured into water and extracted with ethyl acetate. The organic extracts were combined, dried over sodium sulfate and concentrated under reduced pressure. The residue was subjected to silica gel chromatography, eluting with hexane containing from 0-20% ethyl acetate. Fractions containing product were combined and concentrated under reduced pressure to provide 0.20 gm (79%) of the title compound.

Preparation VIII

Alternate Synthesis of 1-benzyl-3,3-dimethyl-4-oxopiperidine

Benzylamine (214 g, 2 mol) is combined with formaldehyde (37% in water, 375 g, 4.5 mol) in ethanol (1 L) with occasional cooling. This biphasic mixture is added over a period of 90 minutes to a refluxing solution of 2-methyl-3-butanone (182 g, 2.11 mol) in anhydrous ethanol (1L) and hydrochloric acid (209 g of 37% solution, 2.1 mol). The brownish solution is heated at reflux for an additional 18 hours. Then triethylamine (310 mL, 223.8 g, 2.21 mol) and formaldehyde (50 g, 36%, 0.6 mol) are added sequentially and the reaction mixture is heated at reflux for 24 hours. The reaction mixture is then cooled to 5°C and treated with potassium hydroxide (117.6 g, 2.1 mol, dissolved in 200 mL of water). The reaction mixture is then extracted with heptane (2 X 500 mL) and methyl tert-butyl ether (2 X 500 mL). The organic extracts is then combined, dried over anhydrous sodium sulfate, filtered and concentrated under vacuum to provide the title compound, (339.36 g after 18% by volume of the above combined organic extracts was removed prior to concentration). This material was purified by chromatography on silica gel (methylene chloride/ethanol, 100:1) to provide purified title compound. $^1$H NMR ($CDCl_3$) δ 1.14 (s, 6H), 2.41 (s,2H), 2.52 (t, 2H), 2.72 (t, 2H), 3.57 (s, 2H), 7.2-7.4 (m, 5H).

Preparation IX

Alternate Synthesis of 1-benzyl-3,3-dimethyl-4-oxopiperidine

In a 1 liter 3-necked flask equipped with mechanical stirring, addition funnel and a calcium chloride drying tube is added a 37% weight solution of formaldehyde (168.5 mL, 2.25 mole) dissolved in 500 mL of absolute ethanol. The resulting solution is cooled in an ice-water bath to 10°C, and benzylamine (109 mL, 1mole) is added dropwise over a one hour period. In a separate 3 liter 3-necked flask equipped with mechanical stirring, addition funnel and two condensers is added 3-methyl-2-butanone (113 mL, 1.06mole) dissolved in 500ml of absolute ethanol and concentrated

hydrogen chloride (92 mL, 1.11mole). The resulting solution is brought to reflux and the formaldehyde/benzylamine solution is added dropwise over a 2 hour period. This solution is refluxed overnight, and then cooled to ambient temperature. Diisopropylethylamine (142.2 g, 1.1 mole) and formaldehyde (22.46 mL, 0.3mole) are added and the resulting solution is heated to reflux for six hours, and then cooled to ambient temperature. The solution is quenched with potassium hydroxide (61.6 g, 1.1 mole) in 200 mL of water, and then extracted with 500 mL ethyl acetate three times. The organics are concentrated under vacuum to give 225 g of red oil. The crude oil is dissolved in 1 liter of methylene chloride. This solution is carefully poured over 1 kg of silica gel on a sintered glass filter. The silica gel is washed with 4 L of methylene chloride. The methylene chloride is concentrated under vacuum to provide 142 g of a yellow oil which crystallizes in the freezer overnight. Yield=65.4%.

Preparation X

cis-1-benzyl-3,5-dimethyl-4-oxopiperidine

A solution of 45 mL (90.4 mMol) lithium diisopropylamide (2.0 M in tetrahydrofuran) was cooled to -5°C. A solution of 18.5 gm (75.4 mMol) 1-benzyl-3-methyl-4-oxopiperidine N,N-dimethylhydrazone (prepared from 1-benzyl-3-methyl-4-oxopiperidine and N,N-dimethylhydrazine) in 100 mL tetrahydrofuran was added dropwise over 40 minutes. The reaction mixture was then cooled to -78°C and 5.16 mL (83 mMol) iodomethane were added dropwise. The reaction mixture was stirred for about 16 hours, warming gradually to room temperature. The resulting homogeneous yellow solution was diluted with 300 mL dichloromethane and was washed first with 100 mL water and then with saturated aqueous sodium chloride. The organic phase was dried over magnesium sulfate and then concentrated under reduced pressure.

This compound (8.5 gm) was dissolved in 200 mL 2.0 M methanolic hydrogen chloride and the resulting mixture heated at reflux for about 16 hours. The reaction mixture was concentrated under reduced pressure and the residue partitioned between 80 mL 5N sodium hydroxide and 200 mL ethyl acetate. The aqueous phase was extracted 3 x 400 mL ethyl acetate. The organic phases were combined, dried over magnesium sulfate, and concentrated under reduced pressure. The residue was subjected to silica gel chromatography, eluting with a gradient of hexane containing from 0-20% ethyl acetate. Fractions containing the desired compound were combined and concentrated under reduced pressure to provide 5.2 gm of the title compound.
Ion Spray MS: m/e = 218 (M+1)

Preparation XI

2-trimethylsilyl-7-bromobenzothiphene

A solution of 6.58 mL (47 mMol) diisopropylamine in 25 mL tetrahydrofuran was cooled to 0°C and then 29.4 mL (47 mMol) n-butyllithium (1.6 M in hexanes) were added. The mixture was allowed to warm to room temperature over 1 hour and was then added dropwise to a mixture of 5.0 gm (24 mMol) 7-bromobenzothiophene and 6.1 mL (47 mMol) trimethylsilyl chloride in 15 mL tetrahydrofuran at -78°C. The reaction mixture was stirred for 2 hours and was then poured into ice water. This mixture was extracted well with diethyl ether. The combined organic phases were washed with 1N hydrochloric acid, dried over sodium sulfate and concentrated under reduced pressure to provide 6.1 gm (91%) of the title compound.

Preparation XII

2-trimethylsilyl-5-fluoro-7-bromobenzothiphene

Beginning with 2 gm (8.6 mMol) 5-fluoro-7-bromobenzothiophene, 0.87 gm (33%) of the title compound were prepared essentially as described in Preparation XI.
$^1$H-NMR($CDCl_3$): δ 7.19 (dd, 1H), 7.05 (dd, 1H), 7.03 (s, 1H), 0.16 (s, 9H).

Preparation XIII

1-(tert-butoxycarbonyl)-3,3-dimethyl-4-oxopiperidine

A mixture of 5.0 gm (23 mMol) 1-benzyl-3,3-dimethyl-4-oxopiperidine, 2.5 gm 5% palladium on carbon, 5.0 gm (23 mMol) di(tert-butyl) dicarbonate in 100 mL ethanol was pressurized to 50 p.s.i. with hydrogen and warmed to 50°C. After 18 hours the reaction mixture was filtered through a bed of celite and the filtrate was concentrated under

reduced pressure. The residue was subjected to silica gel chromatography, eluting with hexane containing 20% ethyl acetate. Fractions containing product were combined and concentrated under reduced pressure to provide 2.9 gm (56%) of the title compound as a white solid.
$^1$H-NMR($CDCl_3$) : δ 3.70 (br s 2H), 3.40 (br s 2H), 2.43 (t, 2H), 1.45 (s, 9H), 1.05 (s, 6H).

Preparation XIV

1-(tert-butoxycarbonyl)-2-methyl-4-trifluoromethanesulfonyloxy-1,2,3,6-tetrahydropyridine

A solution of 5 mL (49 mMol) 4-methoxypyridine in 200 mL tetrahydrofuran was cooled to -40°C, and then 6.9 mL (55 mMol) phenyl chloroformate were added dropwise. After stirring for 15 minutes, 20 mL (60 mMol) methyl magnesium chloride (3M in tetrahydrofuran) were added dropwise and the reaction mixture was allowed to warm to room temperature. After stirring for 30 minutes, the reaction mixture was cooled to -40°C and treated with 340 mMol potassium tert-butoxide. The reaction mixture was allowed to warm to room temperature. After stirring for 1 hour, the reaction mixture was cooled to -40°C and was treated with. 200 mL saturated aqueous oxalic acid. The reaction was warmed to 20°C and allowed to stir for 1 hour. The mixture was extracted 2 x 200 mL diethyl ether. The combined organic phases were washed sequentially with 4 x 100 mL 0.5 N sodium hydroxide, 2 x 100 mL saturated aqueous sodium bicarbonate, 3 x 100 mL deionized water, and 100 mL saturated aqueous sodium chloride. The remaining organics were dried over magnesium sulfate and concentrated under reduced pressure. The residue was subjected to silica gel chromatography, eluting with hexanes containing 40% ethyl acetate. Fractions containing product were combined and concentrated under reduced pressure to provide 4.9 gm (47%) 1-(tert-butoxycarbonyl)-2-methyl-4-oxopiperidine.
EA: Calculated for: $C_{11}H_{17}NO_3$: C, 62.54; H, 8.11; N, 6.63. Found: C, 62.78; H, 8.08; N, 6.76.

A solution of 1.65 gm (7.81 mmol) 1-(tert-butoxycarbonyl)-2-methyl-4-oxopiperidine in 20 mL tetrahydrofuran was cooled to -40°C and was then treated with 8.59 mL (8.59 mMol) lithium tri(sec-butyl)borohydride (1M in tetrahydrofuran). After stirring for 2 hours, the solution was treated with 3.37 gm (8.59 mMol) 2-[N,N-bis(trifluoromethylsulfonyl) amino]-5-chloropyridine and the solution was allowed to warm to room temperature. After stirring for 1 hour, the reaction was diluted with 250 ml diethyl ether and filtered through celite. The celite pad was rinsed with 250 mL diethyl ether and the combined filtrates concentrated under reduced pressure. The residue was subjected to silica gel chromatography, eluting with hexanes containing from 0-9% ethyl acetate. Fractions containing product were combined and concentrated under reduced pressure to provide 2.02 gm (75%) of the title compound.
ISMS: m/e = 346 (M+H)

Preparation XV

1-(tert-butoxycarbonyl)-2-methyl-4-oxopiperidine

A solution of 160.0 gm (733 mMol) di(tert-butyl) dicarbonate in 300 mL tetrahydrofuran was added dropwise to a solution of 100.0 gm (698 mMol) 1,4-dioxa-8-azaspiro-[4,5]decane in 800 mL tetrahydrofuran over 1 hour. The reaction mixture was stirred for 30 minutes after the addition was complete and was then concentrated under reduced pressure. The residue was dissolved in 600 mL diethyl ether and was washed sequentially with 2 x 250 mL deionized water, 2 x 250 mL 5% aqueous sodium bicarbonate, and 250 mL saturated aqueous sodium chloride. The organic phase was dried over potassium carbonate and concentrated under reduced pressure to provide 173.3 gm 8-(tert-butoxycarbonyl)-1,4-dioxa-8-azaspiro[4,5]decane.

A solution of 76.0 gm (312 mMol) 8-(tert-butoxycarbonyl)-1,4-dioxa-8-azaspiro[4,5]decane in 760 mL diethyl ether was cooled to -78°C and was treated with freshly distilled 49.5 mL (328 mMol) N,N,N',N'-tetramethylethylenediamine. One equivalent of a sec-butyllithium solution was added dropwise over 1.5 hours, maintaining the temperature of the reaction mixture below -70°C. After stirring for 4 hours at -78°C, 38.9 mL (625 mMol) iodomethane were added over 10 minutes. The reaction mixture was stirred for 10 minutes and was then allowed to warm gradually to room temperature. The reaction mixture was treated with 300 mL deionized water and the phase were separated. The aqueous phase was extracted with 300 mL diethyl ether. The organic phases were combined, washed with 4 x 250 mL deionized water, dried over potassium carbonate and concentrated under reduced pressure. The residue was subjected to silica gel chromatography, eluting with a gradient of hexanes containing from 7-20% ethyl acetate. Fractions containing product were combined and concentrated under reduced pressure to provide 57.1 gm (71%) 7-methyl-8-(tert-butoxycarbonyl)-1,4-dioxo-8-azaspiro[4,5]decane as a clear oil. This material was cooled to 0-5°C and 279.2 mL trifluoroacetic acid were added. After stirring for 10 minutes, 5.2 mL deionized water were added and the reaction mixture was heated at reflux for 2.5 hours. The reaction mixture was cooled to room temperature and concentrated under reduced pressure. The residue was dissolved in 60 mL ethyl acetate and 120 mL diethyl ether were added over 30

minutes with stirring. The suspension was held in a freezer for 2 hours, filtered, and the solids washed with 30 mL cold diethyl ether to provide 23.5 gm (71.3%) 2-methyl-4-oxopiperidine as a white solid.

A mixture of 12.4 gm (55 mMol) 2-methyl-4-oxopiperidine, 6.89 gm (82 mMol) sodium bicarbonate, and 13.1 gm (60 mMol) di(tert-butoxy) dicarbonate in 40 mL water and 100 mL chloroform was stirred at room temperature for 16 hours. The mixture was diluted with 25 mL deionized water and the phases were separated. The aqueous phase was extracted with 4 x 25 mL chloroform. The organic phases were combined, dried over sodium sulfate, and concentrated under reduced pressure. The residue was subjected to silica gel chromatography, eluting with 3:1 hexane:ethyl acetate containing 1% triethylamine. Fractions containing product were combined and concentrated under reduced pressure to provide 12.0 gm of the title compound as a slightly yellow oil.

Preparation XVI

1-(tert-butoxycarbonyl)-2-ethyl-4-oxopiperidine

Beginning with 1,4-dioxa-8-azaspiro-[4,5]decane and iodoethane, the title compound was prepared essentially as described in Preparation XLII.
ISMS: m/e = 228 (M+1)

Preparation XVII

1-tert-butoxycarbonyl-3,3-dimethyl-4-trifluoromethanesulfonyloxy-1,2,3,6-tetrahydropyridine

A mixture of 10.14 gm (46.66 mMol) 1-benzyl-3,3-dimethyl-4-oxopiperidine, 1.03 gm 10% palladium on carbon, and 11.09 gm (50.81 mMol) di-tert-butyl dicarbonate in 210 mL methanol was purged 3 times with nitrogen and 3 times with hydrogen. The mixture was placed under 50 psig of hydrogen and was shaken at room temperature for 16 hours. The reaction mixture was filtered through a bed of celite and glass microfiber paper. The filtrate was concentrated under reduced pressure and the residue subjected to silica gel chromatography, eluting with hexanes containing 20% ethyl acetate. Fractions containing the product were combined and concentrated under reduced pressure to provide 9.38 gm (88.5%) of 1-(tert-butoxycarbonyl)-3,3-dimethyl-4-oxopiperidine.

A solution of 13.25 mL (26.5 mMol) lithium diisopropylamide (2M in tetrahydrofuran/heptane) was cooled to -78°C. A solution of 5.24 gm (23.05 mMol) of 1-(tert-butoxycarbonyl)-3,3-dimethyl-4-oxopiperidine in 40 mL tetrahydrofuran was added dropwise over 40 minutes, maintaining the reaction temperature below -71°C. After the addition was complete, the reaction mixture was stirred at -78°C for 90 minutes, and then a solution of 8.70 gm (24.35 mMol) N-phenyltrifluoromethanesulfonimide in 40 mL tetrahydrofuran was added over 10 minutes. The solution was allowed to warm to 0°C and was stirred for 90 minutes. The reaction mixture was concentrated under reduced pressure and the residue was subjected to neutral alumina chromatography, eluting with 5:1 hexanes:ethyl acetate. The solvent was removed under reduced pressure and the residue was subjected to silica gel chromatography, eluting with 4:1 hexanes: ethyl acetate. Fractions containing product were combined and concentrated under reduced pressure to provide 1.72 gm (75.2%) of the title compound as a clear, colorless oil.

Alternatively, the lithium diisopropylamide was generated in situ by reacting a solution of 0.86 µL (6.14 mMol) diisopropylamine in 10 mL tetrahydrofuran with 2.2 mL (5.5 mMol) n-butyllithium (2.5 M in hexanes). After cooling this solution to -78°C, a solution of 1.076 gm (4.73 mMol) 1-(tert-butoxycarbonyl)-3,3-dimethyl-4-oxopiperidine in 12 mL tetrahydrofuran was added dropwise, maintaining the reaction temperature below -70°C. After the addition was complete, the reaction mixture was stirred at -78°C for 1 hour, and then a solution of 1.963 gm (5.00 mMol) 2-[N,N-bis (trifluoromethylsulfonyl)amino]-5-chloropyridine in 8 mL tetrahydrofuran was added over 5 minutes. The solution was allowed to warm to 0°C and was stirred for 90 minutes. The reaction mixture was concentrated under reduced pressure and the residue was subjected to neutral alumina chromatography, eluting with 5:1 hexanes:ethyl acetate. The solvent was removed under reduced pressure to provide 1.566 gm (92.1%) of the title compound as a slightly yellow oil.

Preparation XVIII

(6-fluorobenzothien-7-yl)boronic acid

A solution of 4.25 mL (6.8 mMol) n-butyllithium (1.6 M in hexane) in 12 mL tetrahydrofuran was cooled to -78°C. To this solution was added a solution of 1.494 gm (6.46 mMol) 6-fluoro-7-bromobenzothiophene in 17 mL tetrahydrofuran over 35 minutes, maintaining the temperature less than -72°C. The reaction mixture was stirred for 40 minutes and then 810 µL (7.3 mMol) trimethylborate were added over 20 minutes. The reaction mixture was allowed to warm to 0°C, and was held at this temperature for 1.5 hours. The reaction mixture was then treated with 7.5 mL 1N

hydrochloric acid. The mixture was stirred for 15 minutes and the phases were separated. The aqueous phase was extracted well with diethyl ether. The organic phases were combined, dried over sodium sulfate, and concentrated under reduced pressure. The residue was slurried in 15 mL hexanes for 1 hour, the solvent removed by decantation, and the solids dried under reduced pressure to provide 1.234 gm of the title compound as a white solid.

Preparation XIX

6-fluoro-7-bromobenzothiophene

1-(methoxymethoxy)-3-fluorobenzene

A solution of 100 gm (0.892 mol) 3-fluorophenol in 800 mL dichloromethane was cooled to -10°C. To this solution were added 155 gm (1.20 mol) diisopropylethylamine dropwise over 15 minutes. Residual diisopropylethylamine in the addition funnel was rinsed into the reaction mixture with 100 mL dichloromethane. To the resulting solution were added 100 gm (1.24 mol) chloromethyl methyl ether over 5 minutes, resulting an exotherm from -10°C to 2°C. The reaction mixture was allowed to warm to room temperature. After 22 hours the reaction mixture was quenched with 500 mL water. The organic phase was washed with 500 mL 2M aqueous sodium bisulfate followed by 300 mL 1M sodium hydroxide. The remaining organics were dried over magnesium sulfate and concentrated under reduced pressure to provide 130 gm of a yellow oil. The oil was distilled (kugelrohr, 80°C, 0.1 torr) to provide 126 gm (91%) of the desired compound as a colorless oil.

1-(methoxymethoxy)-2-bromo-3-fluorobenzene

A solution of 270 mL (430 mMol) n-butyllithium (1.6 M in hexanes) in 800 mL tetrahydrofuran was cooled to -78°C. To this solution was added a solution of 60 gm (384 mMol) 1-(methoxymethoxy)-3-fluorobenzene in 100 mL tetrahydrofuran over 30 minutes. The resulting mixture was stirred for 1 hour and was then rapidly quenched with 67.5 gm (423 mMol) bromine. The reaction mixture was stirred for 30 minutes and was then partitioned by the addition of 500 mL methyl tert-butyl ether and 300 mL saturated aqueous sodium thiosulfate. The organic phase was dried over sodium sulfate and concentrated under reduced pressure. The residual oil was distilled (kugelrorh, 70-100°C, 0.1 torr) to provide 81.7 gm (91%) of the desired compound as a colorless oil.

2-bromo-3-fluorophenol

A mixture of 120 mL isopropanol, 200 mL 6M hydrochloric acid, and 81 gm (345 mMol) 1-(methoxymethoxy)-2-bromo-3-fluorophenol was stirred at room temperature. After 2.5 hours the reaction mixture was partitioned by the addition of 400 mL methyl tert-butyl ether and 120 mL 5N sodium hydroxide. The organic phase was washed with saturated aqueous sodium chloride, dried over sodium sulfate and concentrated under reduced pressure. The residual oil was subjected to silica gel chromatography, eluting with dichloromethane. Fractions containing product were combined and concentrated under reduced pressure to provide 55.1 gm (84%) of the desired compound as a colorless oil.

O-(3-fluoro-2-bromophenyl)-N,N-diethylthiocarbamate

A solution of 54.7 gm (286 mMol) 2-bromo-3-fluorophenol and 19.3 gm (292 mMol) 85% sodium hydroxide in 200 mL water was cooled to 5°C and was then treated with a solution of 46.0 gm (372 mMol) N,N-diethylthiocarbonyl chloride in 150 mL tetrahydrofuran over 30 minutes. After 20 minutes the reaction mixture was partitioned by the addition of 200 mL methyl tert-butyl ether and 30 mL 5N sodium hydroxide. The aqueous phase was extracted with 100 mL methyl tert-butyl ether followed by 100 mL dichloromethane. The organic phases were combined, washed with 50 mL 1M sodium hydroxide followed by saturated aqueous sodium chloride, dried over sodium sulfate and concentrated under reduced pressure to provide 98 gm of a yellow solid. The solid was combined with 300 mL heptane and was stirred for 10 minutes. The suspension was filtered and dried to provide 73.4 gm of the desired compound as a white powder.

S-(3-fluoro-2-bromophenyl)-N,N-diethylthiocarbamate

A mixture of 69.25 gm (226 mMol) O-(3-fluoro-2-bromophenyl)-N,N-diethylthiocarbamate and 70 mL diphenyl ether was stirred at 240°C for 45 minutes. The reaction mixture was then cooled to 40°C and diluted with 80 mL heptane. The mixture was poured directly onto a column containing 1 kg of silica gel and was eluted with dichloromethane. Fractions containing product were combined and concentrated under reduced pressure to provide 65.9 gm (95%)

of the desired compound as an oil.

2-(3-fluoro-2-bromophenylthio)acetaldehyde diethylacetal

A solution of 64 gm (209 mMol) S-(3-fluoro-2-bromophenyl)-N,N-diethylthiocarbamate in 800 mL methanol was treated with 80 gm (1.21 mMol) 85% potassium hydroxide. The addition resulted in an exotherm from 22°C to 53°C over 10 minutes. The reaction mixture was maintained at 50°C for 2 hours and then 91.7 gm (465 mMol) 2-bromoacetaldehyde diethyl acetal were added. After 22 hours the reaction mixture was cooled to room temperature and was then partitioned by the addition of 800 mL methyl tert-butyl ether and 800 mL water. The organic phase was washed with 200 mL 2M aqueous sodium bisulfate, dried over sodium sulfate, and concentrated under reduced pressure. The residue was used without further purification.

Ring closure

A mixture of 60.0 gm (185 mMol) 2-(3-fluoro-2-bromophenylthio)acetaldehyde diethyl acetal and 30 gm AMBERLYST-15™ in 500 mL chlorobenzene was stirred at 120°C. After 3 hours the mixture was cooled, the resin filtered, and the filtrate concentrated under reduced pressure. The residual oil was subjected to silica gel chromatography, eluting with heptane. Fractions containing product were combined and concentrated under reduced pressure to provide 20.8 gm (45.5%) of the title compound as an oil.

EXAMPLE 1

3-methyl-4-(benzothien-7-yl)-1,2,3,6-tetrahydropyridine hydrochloride and 3-methyl-4-(benzothien-7-yl)-1,2,5,6-tetrahydropyridine oxalate

1-benzyl-3-methyl-4-hydroxy-4-(2-trimethylsilylbenzothien-7-yl)piperidine

A solution of 8.1 gm (28 mMol) 2-trimethylsilyl-7-bromobenzothiophene in 60 mL tetrahydrofuran was cooled to -78°C. This solution was then treated with 23.1 mL (37 mMol) n-butyllithium (1.6 M in hexane) and stirred for 15 minutes. A solution of 5.18 gm (25 mMol) 1-benzyl-3-methyl-4-oxopiperidine in 20 mL tetrahydrofuran was then added dropwise. The reaction mixture was stirred for about 20 hours, gradually warming to room temperature. The reaction mixture was then diluted with water and extracted well with ethyl acetate. The organic extracts were combined, dried over sodium sulfate, and concentrated under reduced pressure. The residue was subjected to silica gel chromatography, eluting with 20% ethyl acetate in hexane. Fractions containing the desired compound were combined and concentrated under reduced pressure to provide 5.1 gm (50%).

1-benzyl-3-methyl-4-(benzothien-7-yl)-1,2,3,6-tetrahydropyridine and 1-benzyl-3-methyl-4-(benzothien-7-yl)-1,2,5,6-tetrahydropyridine

A mixture of 5.1 gm (12.5 mMol) 1-benzyl-3-methyl-4-hydroxy-4-(2-trimethylsilylbenzothien-7-yl)piperidine and 5.93 gm (31 mMol) p-toluenesulfonic acid monohydrate was heated at reflux for about 3 hours. The reaction mixture was cooled to room temperature and diluted with ethyl acetate. This mixture was washed 2 x 100 mL 2N sodium hydroxide, dried over sodium sulfate and concentrated under reduced pressure. The residue was subjected to silica gel chromatography, eluting with 20% ethyl acetate in hexane.

Fractions containing 1-benzyl-3-methyl-4-(benzothien-7-yl)-1,2,3,6-tetrahydropyridine were combined and concentrated under reduced pressure to provide 1.37 gm (34.3%).

Fractions containing 1-benzyl-3-methyl-4-(benzothien-7-yl)-1,2,5,6-tetrahydropyridine were combined and concentrated under reduced pressure to provide 0.11 gm (2.6%).

Later fractions were combined and concentrated under reduced pressure to provide 1.96 gm (47%) 1-benzyl-3-methyl-4-hydroxy-4-(benzothien-7-yl)piperidine.

Debenzylation/Salt formation

A solution of 1.37 gm (4.3 mMol) 1-benzyl-3-methyl-4-(benzothien-7-yl)-1,2,3,6-tetrahydropyridine in 20 mL dichloromethane was cooled to 0°C. This solution was treated with 0.92 mL (8.56 mMol) 1-chloroethyl chloroformate dropwise. The reaction mixturer was stirred for two hours, warming gradually to room temperature. The reaction mixture was then concentrated under reduced pressure and the residue dissolved in 20 mL methanol. This solution was heated at reflux for 2 hours and was then concentrated under reduced pressure. This residue was subjected to silica gel

chromatography. Fractions containing product were combined and concentrated under reduced pressure to provide 0.73 gm 3-methyl-4-(benzothien-7-yl)-1,2,3,6-tetrahydropyridine. This material was treated with hydrogen chloride in diethyl ether to provide 3-methyl-4-(benzothien-7-yl)-1,2,3,6-tetrahydropyridine hydrochloride.
m.p. = 150-155°C

Beginning with 0.10 gm (0.31 mMol) 1-benzyl-3-methyl-4-(benzothien-7-yl)-1,2,5,6-tetrahydropyridine, 0.032 gm 3-methyl-4-(benzothien-7-yl)-1,2,5,6-tetrahydropyridine was prepared as described in the previous paragraph. This material was treated with oxalic acid to provide 3-methyl-4-(benzothien-7-yl)-1,2,5,6-tetrahydropyridine oxalate.
m.p. = 201-203°C
EA: Calculated for: $C_{14}H_{15}NS$-$C_2H_2O_4$: C, 60.17; H, 5.37; N, 4.39. Found: C, 59.94; H, 5.49; N, 4.62.

EXAMPLE 2

3,3-dimethyl-4-(benzothien-7-yl)-1,2,3,6-tetrahydropyridine hydrochloride

Beginning with 2-trimethylsilyl-7-bromobenzothiophene and 1-benzyl-3,3-dimethyl-4-oxopiperidine, the title compound was prepared essentially as described in EXAMPLE 1. m.p. = 204-206°C
EA: Calculated for: $C_{15}H_{17}NS$-HCl: C, 64.38; H, 6.48; N, 5.00; S, 11.46. Found: C, 64.70; H, 6.55; N, 5.23; S, 11.55.

EXAMPLE 3

3,3-dimethyl-4-(4-fluorobenzothien-7-yl)-1,2,3,6-tetrahydropyridine hydrochloride

Beginning with 2-trimethylsilyl-4-fluoro-7-bromobenzothiophene and 1-benzyl-3,3-dimethyl-4-oxopiperidine, the title compound was prepared essentially as described in EXAMPLE 1.
m.p. = 240-242°C
Ion Spray MS: m/e = 262 (M+1)
High Resolution MS: Theory: 262.1065. Found: 262.1074.

EXAMPLE 4

cis-3-methyl-4-(6-fluorobenzothien-7-yl)piperidine oxalate

1-benzyl-3-methyl-4-hydroxy-4-(2-trimethylsilyl-6-fluorobenzothien-7-yl)piperidine

A solution of 1.12 gm (5.0 mMol) 2-trimethylsilyl-6-fluorobenzothiophene in 5.6 mL tetrahydrofuran was cooled to - 78°C. A solution of 3.2 mL (5.5 mMol) tert-butyllithium (1.7 M in pentane) was added dropwise over 20 minutes. Once the addition was complete, the reaction mixture was stirred for 1 hour and then a solution of 1.12 gm (5.5 mMol) 1-benzyl-3-methyl-4-oxopiperidine in 3.6 mL tetrahydrofuran was added dropwise over 10 minutes. The cooling bath was removed and the reaction mixture was stirred for 1 hour, gradually warming to room temperature. The reaction mixture was then treated with 5 mL saturated aqueous ammonium chloride followed by 5 mL water. This mixture was extracted with 3 x 25 mL ethyl acetate. The organic extracts were combined, washed with saturated aqueous sodium chloride, dried over sodium sulfate and concentrated under reduced pressure. The residue gradually began to crystallize. The crystalline mass was dissolved in 25 mL diethyl ether and was heated at reflux. To this heated solution were added 30 mL hexane, and the mixture reduced in volume until most of the diethyl ether was removed. The solution was then cooled to room temperature. The crystals which formed were collected by filtration to provide 1.10 gm of the desired compound.
EA: Calculated for: $C_{24}H_{29}NOSFSi$: C, 67.41; H, 7.07; N, 3.28. Found: C, 67.51; H, 7.01; N, 3.42.

1-benzyl-3-methyl-4-(methyl oxoacetoxy)-4-(2-trimethylsilyl-6-fluorobenzothien-7-yl)piperidine

A solution of 0.47 gm (1.15 mMol) 1-benzyl-3-methyl-4-hydroxy-4-(2-trimethylsilyl-6-fluorobenzothien-7-yl) piperidine and 0.42 gm (3.45 mMol) 4-(dimethylamino)pyridine in 3 mL dichloromethane was cooled in an ice water bath. To this mixture were added 0.32 mL (3.45 mMol) methyl chlorooxoacetate and the resulting mixture was stirred for 7 hours at room temperature. The reaction mixture was poured into a mixture of 15 mL dichloromethane and 15 mL ice water. The phases were separated and the aqueous phase was extracted with 20 mL dichloromethane. The combined organic phases were washed with saturated aqueous sodium chloride, dried over sodium sulfate, and concentrated under reduced pressure to provide a white solid.

cis-1-benzyl-3-methyl-4-(2-trimethylsilyl-6-fluorobenzothien-7-yl)piperidine

A solution of 0.57 gm (1.15 mMol) 1-benzyl-3-methyl-4-(methyl oxoacetoxy)-4-(2-trimethylsilyl-6-fluorobenzothien-7-yl)piperidine, 0.62 mL (2.3 mMol) tri(n-butyl)tin hydride, and 0.10 gm (0.58 mMol) 2,2'-azobisisobutyronitrile in 6 mL of toluene was stirred at reflux for about 2.5 hours. The reaction mixture was concentrated under reduced pressure and the residue subjected to radial silica gel chromatography (4 mm plate), eluting first with hexane and then with 25% ethyl acetate in hexane. Fractions containing the desired compound were combined and concentrated under reduced pressure to provide 0.157 gm (34%).

cis-1-benzyl-3-methyl-4-(6-fluorobenzothien-7-yl)piperidine

A mixture of 0.15 gm (0.36 mMol) cis-1-benzyl-3-methyl-4-(2-trimethylsilyl-6-fluorobenzo-thien-7-yl)piperidine and 0.20 gm (1.07 mMol) p-toluenesulfonic acid monohydrate in 20 mL toluene was heated at 100°C for 3 hours. The reaction mixture was then cooled to room temperature and diluted with 50 mL ethyl acetate. This mixture was washed sequentially with 3 x 25 mL 0.2 N sodium hydroxide and 25 mL saturated aqueous sodium chloride, dried over sodium sulfate and concentrated under reduced pressure. The residue was subjected to radial silica gel chromatography (2 mm plate), eluting with 25% ethyl acetate in hexane. Fractions containing product were combined and concentrated under reduced pressure to provide 0.11 gm of the desired compound.

Debenzylation/salt formation

Beginning with 0.11 gm (0.30 mMol) cis-1-benzyl-3-methyl-4-(6-fluorobenzothien-7-yl)piperidine, 0.080 gm of cis-3-methyl-4-(6-fluorobenzothien-7-yl)piperidine were prepared essentially as described in EXAMPLE 1. Treatment of this compound with oxalic acid in acetone provided 0.058 gm of the title compound.
Ion Spray MS: m/e = 250 (M+1)
EA: Calculated for: $C_{14}H_{16}NSF-C_2H_2O_4$: C, 56.62; H, 5.35; N, 4.13. Found: C, 56.61; H, 5.54; N, 3.92.

EXAMPLE 5

cis-3-ethyl-4-(benzothien-7-yl)piperidine hydrochloride

1-benzyl-3-methyl-4-hydroxy-4-(2-trimethylsilylbenzothien-7-yl)piperidine

Beginning with 1.4 gm (4.9 mMol) 2-trimethylsilyl-7-bromobenzofuran and 1.0 gm (4.7 mMol) 2-benzyl-3-ethyl-4-oxopiperidine, 0.67 gm (37%) of the desired compound were prepared essentially as described in EXAMPLE 4.
Ion Spray MS: m/e = 424 (M+1)
EA: Calculated for: $C_{24}H_{30}NOSSi$: C, 70.87; H, 7.85; N, 3.30. Found: C, 71.20; H, 7.99; N, 3.33.

cis-1-benzyl-3-ethyl-4-(2-trimethylsilylbenzothien-7-yl)piperidine

Beginning with 0.65 gm 1-benzyl-3-ethyl-4-hydroxy-4-(2-trimethylsilylbenzothien-7-yl)piperidine, 0.72 gm 1-benzyl-3-ethyl-4-(methyl oxoacetoxy)-4-(2-trimethylsilylbenzothien-7-yl)piperidine were prepared essentially as described in EXAMPLE 4.
Ion Spray MS: m/e = 510 (M+1)
Beginning with 0.71 gm 1-benzyl-3-ethyl-4-(methyl oxoacetoxy)-4-(2-trimethylsilylbenzothien-7-yl)piperidine, 0.20 gm of the desired compound were prepared essentially as described in EXAMPLE 4.
Ion Spray MS: m/e = 408 (M+1)

cis-3-ethyl-4-(2-trimethylsilylbenzothien-7-yl)piperidine

Beginning with 0.19 gm (0.56 mMol) cis-1-benzyl-3-methyl-4-(2-trimethylsilylbenzothien-7-yl)piperidine, 0.10 gm (68%) of the desired compound were prepared essentially as described in EXAMPLE 4.

Desilylation/salt formation

Beginning with 0.10 gm (0.31 mMol) cis-3-ethyl-4-(2-trimethylsilylbenzothien-7-yl)piperidine, 0.05 gm (56%) of the title compound were prepared as an off-white powder essentially as described in EXAMPLE 4.

EXAMPLE 6

cis-3-methyl-4-(5-fluorobenzothien-7-yl)piperidine hydrochloride

1-benzyl-3-methyl-4-hydroxy-4-(5-fluorobenzothien-7-yl)piperidine

A suspension of 0.24 gm (10 mMol) magnesium turnings in 20 mL tetrahydrofuran was sonicated under nitrogen for 10 minutes. A solution of 1.85 gm (8 mMol) 5-fluoro-7-bromobenzothiophene in 5 mL tetrahydrofuran was added followed by two drops of 1,2-dibromoethane. The mixture was sonicated as temperature rose to 45-50°C. The reaction mixture was stirred for about 1.5 hours and was then cooled in an ice bath. A solution of 2.15 gm (10.6 mMol) 1-benzyl-3-methyl-4-oxopiperidine in 5 ml tetrahydrofuran was then added dropwise. The reaction mixture was allowed to warm to room temperature and was then heated at 40-50°C for about 18 hours. The reaction mixture was cooled in an ice bath and was quenched with saturated aqueous ammonium chloride and ice. This mixture was then extracted well with ethyl acetate. The organic extracts were combined, washed with saturated aqueous sodium chloride, dried over sodium sulfate and concentrated under reduced pressure. The residue was subjected to silica gel chromatography, eluting with a gradient of dichloromethane containing from 0-8% methanol. Fractions containing product were combined and concentrated under reduced pressure to provide 0.78 gm of the desired compound in two portions.
Ion Spray MS: m/e = 356 (M+1)

cis-1-benzyl-3-methyl-4-(5-fluorobenzothien-7-yl)piperidine

Beginning with 0.50 gm 1-benzyl-3-methyl-4-hydroxy-4-(5-fluorobenzothien-7-yl)piperidine, 0.50 gm 1-benzyl-3-methyl-4-(methyl oxoacetoxy)-4-(5-fluorobenzothien-7-yl)-piperidine were prepared essentially as described in EXAMPLE 4.
Ion Spray MS: m/e = 442 (M+1)

Beginning with 0.50 gm 1-benzyl-3-methyl-4-(methyl oxoacetoxy)-4-(5-fluorobenzothien-7-yl)piperidine, 0.20 gm of the desired compound were prepared essentially as described in EXAMPLE 4.
Ion Spray MS: m/e = 408 (M+1)

Debenzylation/salt formation

Beginning with 0.18 gm (0.54 mMol) cis-1-benzyl-3-methyl-4-(5-fluorobenzothien-7-yl)piperidine, 0.080 gm (53%) of the title compound were prepared essentially as described in EXAMPLE 1.
Ion Spray MS: m/e = 250 (M+1)

EXAMPLE 7

cis-3-methyl-4-(benzothien-7-yl)piperidine hydrochloride

Beginning with 7-bromobenzothiophene and 1-benzyl-3-methyl-4-oxopiperidine, the title compound was prepared essentially as described in EXAMPLE 6.
m.p. >270°C
Ion Spray MS: m/e = 232 (M+1)

EXAMPLE 8

3,3-dimethyl-4-(benzothien-7-yl)piperidine oxalate

Beginning with 7-bromobenzothiophene and 1-benzyl-3,3-dimethyl-4-oxopiperidine, the title compound was prepared essentially as described in EXAMPLE 6.
m.p. = 192-195°C
Ion Spray MS: m/e = 246 (M+1)

EXAMPLE 9

3-methyl-4-(benzothien-4-yl)piperidine hydrochloride

Beginning with a mixture of 4-bromobenzothiophene and 6-bromobenzothiophene, and 1-benzyl-3-methyl-4-oxopiperidine, the title compound was prepared essentially as described in EXAMPLE 6. The desired compound was separated by chromatography from the isomeric mixture after the mixture had been debenzylated in the final step of the synthesis.
m.p. >280°C
High Resolution MS: Theory: 232.1160. Found: 232.1172.

EXAMPLE 10

2-methyl-4-(benzothien-7-yl)piperidine hydrochloride

Beginning with 2-(trimethylsilyl)-7-bromobenzofuran and 1-allyl-2-methyl-4-oxopiperidine, 1-allyl-2-methyl-4-(benzothien-7-yl)piperidine was prepared essentially as described in EXAMPLE 4. The allyl group was removed by heating a mixture of 0.166 gm (0.61 mMol) 1-allyl-2-methyl-4-(benzothien-7-yl)piperidine and 0.003 gm tris(triphenyl-phosphine)rhodium(I) chloride in 9 mL ethanol to reflux, removing solvent by distillation. Once 2.5 mL solvent had been removed, an additional 2.5 mL solvent and .0025 gm tris(triphenyl-phosphine)rhodium(I) chloride were added and an additional 2.5 mL solvent were removed by distillation. This cycle was repeated two more times and then the reaction mixture was cooled to room temperature. This cooled mixture was then poured over a Varian SCX column, eluting sequentially with 5 x 20 mL methanol, and 2M ammonium hydroxide in methanol. Fractions containing the desired compound were combined and concentrated under reduced pressure. The residue was subjected to silica gel chromatography, eluting with ethyl acetate containing 10% 2M ammonium hydroxide in methanol. Fractions containing product were combined and concentrated under reduced pressure to provide 0.07 gm (47%) of a tan oil. This oil was dissolved in diethyl ether and treated with. diethyl ether containing hydrogen chloride. The precipitate which formed was collected by filtration and dried to provide 0.5 gm of the title compound as a white powder.
m.p. = 237-238°C
MS: m/e = 232 (M+1)

EXAMPLE 11

3-methyl-4-(4-fluorobenzothien-7-yl)piperidine hydrochloride

Beginning with 2-(trimethylsilyl)-4-fluoro-7-bromobenzothiophene and 1-benzyl-3-methyl-4-oxopiperidine, the title compound was prepared essentially as described in EXAMPLE 4. m.p. >320°C
MS: m/e = 250 (M+1)

EXAMPLE 12

3,3-dimethyl-4-(5-fluorobenzothien-7-yl)-1,2,3,6-tetrahydropyridine fumarate

Beginning with 2-trimethylsilyl-5-fluoro-7-bromobenzothiophene and 1-(tert-butoxycarbonyl)-3,3-dimethyl-4-oxopiperidine, the title compound was prepared essentially as described in EXAMPLE 1.
MS: m/e = 262.

EXAMPLE 13

3,3-dimethyl-4-(6-fluorobenzothien-7-yl)-1,2,3,6-tetrahydropyridine hydrochloride

A solution of 4.35 gm (19.4 mMol) 2-tri.methylsilyl-6-fluorobenzothiophene in 22 mL tetrahydrofuran was cooled to - 78°C. To this cooled solution were added 12.5 mL (21.3 mMol) tert-butyllithium (1.7 M in pentane) dropwise over 70 minutes. The reaction mixture was stirred an additional 15 minutes and then a solution of 4.63 gm (21.3 mMol) 1-benzyl-3,3-dimethylpiperidin-4-one in 13 mL tetrahydrofuran was added dropwise over 20 minutes. Once the addition was complete, the cooling bath was removed and the reaction mixture allowed to warm to room temperature over two hours. The mixture was then treated with 5 mL saturated aqueous ammonium chloride followed by water. The mixture was extracted 2 x 25 mL ethyl acetate. The organic phases were combined, washed with saturated aqueous sodium chloride, dried over magnesium sulfate and concentrated under reduced pressure. The residue was subjected to radial chromatography (silica gel, 4mm plate), eluting with hexane at a rate of 9 mL/minute and then 3:1 acetone:hexane. Fractions containing product were combined and concentrated under reduced pressure to provide 4.13 gm of an oil. This oil was dissolved in 36 mL ethanol and was treated with 1.06 gm sodium borohydride. The mixture was stirred 45 minutes at room temperature and was then quenched with water. The mixture was extracted 2 x 50 mL ethyl acetate.

The organic phases were combined, washed with saturated aqueous sodium chloride, dried over sodium sulfate, and concentrated under reduced pressure. The residue was subjected to silica gel chromatography, eluting with 15% ethyl acetate in toluene. Fractions containing product were combined and concentrated under reduced pressure to provide 3.47 gm (47%) of 3,3-dimethyl-4-hydroxy-4-(6-fluorobenzothien-7-yl)piperidine. MS: m/e = 442 (M+1)

The 3,3-dimethyl-4-hydroxy-4-(6-fluorobenzothien-7-yl)piperidine was converted to the title compound essentially as described in EXAMPLE 1.
ISMS: m/e = 262 (M+1)
EA: Calculated for: $C_{15}H_{16}$FNS-HCl: C, 60.49; H, 5.75; N, 4.70. Found: C, 60.35; H, 5.55; N, 4.65.

EXAMPLE 14

Alternate Synthesis of 3,3-dimethyl-4-(6-fluorobenzothien-7-yl)-1,2,3,6-tetrahydropyridine hydrochloride

A solution of 10.6 gm (29.50 mMol) 1-(tert-butoxycarbonyl)-3,3-dimethyl-4-trifluoromethanesulfonyloxy-1,2,3,6-tetrahydropyridine in 110 mL previously deoxygenated 9:1 toluene:n-propanol was placed under vacuum and pressurized with nitrogen three times to exclude oxygen. To this solution were added 0.23 gm (1.02 mMol) palladium acetate and 0.458 gm (1.75 mMol) triphenylphosphine and the resulting mixture was stirred for 15 minutes. Then 6.3 gm (32.14 mMol) 6-fluorobenzothien-7-ylboronic acid, 1.88 gm (44.35 mMol) lithium chloride, and 16.2 mL (32.4 mMol) of previously deoxygenated 2.0 M aqueous sodium carbonate were added to the reaction mixture. The mixture was deoxygenated by subjecting it three times to a vacuum/nitrogen cycle, was heated to reflux for 2.5 hours, and was then cooled to room temperature. The reaction mixture was then partitioned between water and diethyl ether. The aqueous phase was extracted well with diethyl ether and all of the organic phases were combined, dried over sodium sulfate and concentrated under reduced pressure. The residue was subjected to silica gel chromatography, eluting with 25% methyl tert-butyl ether in hexanes. Fractions containing product were combined and concentrated under reduced pressure to provide 5.53 gm 1-(tert-butoxycarbonyl)-3,3-dimethyl-4-(6-fluorobenzothien-7-yl)-1,2,3,6-tetrahydropyridine as a white solid. This material was heated in 26.5 mL hexanes and the mixture was then allowed to cool to room temperature. After one hour, the mixture was cooled in an ice bath. After two hours the solid was filtered, washed with cold hexane, and dried under reduced pressure to provide 4.27 gm 1-(tert-butoxycarbonyl)-3,3-dimethyl-4-(6-fluorobenzothien-7-yl)-1,2,3,6-tetrahydroxypyridine as a white solid.
MS: m/e = 361.1512

A solution of 3.447 gm (9.54 mMol) 1-(tert-butoxycarbonyl)-3,3-dimethyl-4-(6-fluorobenzothien-7-yl)-1,2,3,6-tetrahydropyridine in 35 mL 2 M hydrogen chloride in ethyl acetate was stirred at room temperature for 2.5 hours. The reaction mixture was concentrated under reduced pressure to provide 2.93 gm of the title compound as a white solid.

EXAMPLE 15

Trans-2-methyl-4-(benzothien-4-yl)piperidine succinate

1-(tert-butoxycarbonyl)-4-hydroxy-4-(benzothien-4-yl)piperidine

A solution of 10.10 gm (47.4 mMol) of a mixture of 4-bromobenzothiophene and 6-bromothiophene in 200 mL diethyl ether was treated with 2.30 gm (94.8 mMol) magnesium followed by the gradual addition of 4.1 mL (47.4 mMol) 1,2-dibromoethane. The reaction mixture was stirred at reflux for about 2.5 hours and was then cooled to room temperature. To this mixture was then added a solution of 10.39 gm (52.1 mMol) 1-(tert-butoxycarbonyl)piperidin-4-one in diethyl ether. The resulting mixture was stirred at room temperature overnight and was then diluted with saturated aqueous ammonium chloride. The reaction mixture was then extracted well with ethyl acetate. The combined organic extracts were dried over sodium sulfate and concentrated under reduced pressure. The residue was subjected to silica gel chromatography, eluting a gradient of dichloromethane containing from 0-5% methanol. Fractions containing product were combined and concentrated under reduced pressure to provide 3.09 gm (20%) of the desired compound.
MS: m/e = 334 (M+1)

4-(benzothien-4-yl)-1,2,5,6-tetrahydropyridine

A mixture of 3.09 gm (9.25 mMol) 1-(tert-butoxycarbonyl)-4-hydroxy-4-(benzothien-4-yl)piperidine and 5.28 gm (27.8 mMol) p-toluenesulfonic acid in 75 mL toluene was heated at reflux for 4 hours. The reaction mixture was then cooled to room temperature and concentrated under reduced pressure. The residue was partitioned with 2N sodium hydroxide and ethyl acetate. The aqueous phase was extracted well with ethyl acetate and the organic extracts

combined, dried over sodium sulfate, and concentrated under reduced pressure to provide 1.76 gm (88%) of the desired compound.
MS: m/e = 216 (M+1)

4-(benzothien-4-yl)piperidine

A suspension of 1.75 gm (8.12 mMol) 4-(benzothien-4-yl)-1,2,5,6-tetrahydropyridine and 1.8 gm 10% palladium on carbon in 50 mL ethanol was hydrogenated for 2 days at 50°C and 45 p.s.i and 2 additional days at room temperature and 45 p.s.i. After the first day, an additional 2 gm 10% palladium on carbon were added to the reaction mixture. The reaction mixture was filtered and the filtrate concentrated under reduced pressure to provide 0.49 gm (28%) of the desired compound as a clear oil.
MS: m/e = 218 (M+1)

1-chloro-4-(benzothien-4-yl)piperidine

A solution of 0.47 gm (2.17 mMol) 4-(benzothien-4-yl)piperidine in 8 mL tetrahydrofuran and 4 mL diethyl ether was cooled to 0°C. To this solution were added 0.30 gm N-chlorosuccinimide and the reaction mixture was allowed to warm to room temperature. After 1 hour the reaction mixture was concentrated under reduced pressure and the residue partitioned with diethyl ether and aqueous sodium bicarbonate. The phases were separated and the aqueous phase extracted well with diethyl ether. The organic phases were combined, dried over sodium sulfate, and concentrated under reduced pressure to provide 0.41 gm of the desired compound.

Imine formation/methylation

A mixture of 0.38 gm (1.51 mMol) 1-chloro-4-(benzothien-4-yl)piperidine, 0.008 gm 18-crown-6, and 0.28 gm (3.92 mMol) potassium superoxide in 5 mL diethyl ether was stirred at room temperature overnight. The mixture was filtered and the filter cake washed with diethyl ether. The filtrate was added slowly to a solution of 2.7 mL (3.77 mMol) methyllithium (1.4 M in diethyl ether) in 3 mL diethyl ether. The reaction mixture was stirred for 1 hour at room temperature and was then slowly diluted with water. The resulting mixture was extracted well with diethyl ether and then once with ethyl acetate. The combined organic extracts were dried over sodium sulfate and concentrated under reduced pressure. The residue was subjected to silica gel chromatography. Fractions containing product were combined and concentrated under reduced pressure to provide 0.052 gm of trans-2-methyl-4-(benzothien-4-yl)piperidine. The title compound was prepared by treating this material with succinic acid.
MS: m/e = 232 (M+1)

EXAMPLE 15

cis-2-methyl-4-(benzothien-4-yl)piperidine

1-(tert-butoxycarbonyl)-2-methyl-4-hydroxy-4-(benzothien-4-yl)piperidine

Beginning with 25.36 gm 1-(tert-butoxycarbonyl)-2-methylpiperidin-4-one and 23.28 gm (109 mMol) of a mixture of 4-bromobenzothiophene and 6-bromobenzothiophene, 5.17 gm (14%) of the desired compound were prepared as a white foam essentially as described in EXAMPLE 14.
MS: m/e = 348 (M+1)

2-methyl-4-(benzothien-4-yl)-1,2,3,6-tetrahydropyridine

Beginning with 5.13 gm (14.8 mMol) 1-(tert-butoxycarbonyl)-2-methyl-4-hydroxy-4-(benzothien-4-yl)piperidine, 3.13 gm (92%) of the desired compound were prepared essentially as described in EXAMPLE 14.

Reduction

A mixture of 3.115 gm (13.6 mMol) 2-methyl-4-(benzothien-4-yl)-1,2,3,6-tetrahydropyridine and 3.2 gm Royer palladium catalyst in 40 mL methanol was hydrogenated on a Parr shaker at 50°C and 45 p.s.i. for 24 hours. At this point an additional 4 gm of Royer palladium catalyst were added and the hydrogenation continued for an additional 40 hours. The reaction mixture was filtered, the solid washed with methanol, and the combined filtrates concentrated under reduced pressure. The residue was subjected to silica gel chromatography, eluting with a gradient of dichloromethane containing from 0-4% methanol and a trace of ammonia. Fractions containing product were combined and concentrated under reduced pressure to provide 1.46 gm (46%) of cis-2-methyl-4-(benzofur-4-yl)piperidine as a yellow oil.

MS: m/e = 232 (M+1)

The title compound was prepared by treating this material with succinic acid.

The ability of the compounds of this invention to bind to the 5-$HT_{2c}$ receptor subtype was measure essentially as described by Wainscott (Wainscott, et al., Journal of Pharmacology and Experimental Therapeutics, **276**, 720-727 (1996)).

Membrane Preparation

AV12 cells stably transfected with the human 5-$HT_{2c}$ receptors were grown in suspension and harvested by centrifugation, resuspended in 50 mM tris-HCl, pH 7.4, and frozen at -70°C. On the day of assay, an aliquot of cells was thawed, resuspended in 40 mL of 50 mM tris-HCl, pH 7.4, and centrifuged at 39,800 x g for 10 minutes at 4°C. The resulting pellet was resuspended, incubated at 37°C for 10 minutes to remove endogenous serotonin, then centrifuged twice more.

$[^{125}I]$-DOI Binding for Determination of 5-$HT_{2c}$ Receptor Affinity

Briefly, prepared cell membranes were added to dilutions of compounds in a final solution containing 50 mM tris-HCl, pH 7.4, 9.75 mM $MgCl_2$, 0.5 mM EDTA, 10 μM pargyline, 0.1% sodium ascorbate, and 0.1 nM $[^{125}I]$-DOI, with 10 μM mianserin for defining non-specific binding. All incubations (800 μL) were performed at 37°C for 30 minutes before harvesting onto GF/C filters prewet with 0.5% polyethyleneimine, with four 1 mL washes of ice-cold 50 mM tris-HCl, pH 7.4, and counting in a gamma counter. Nonlinear regression analysis was performed on the concentration response curves using a four parameter logistic equation described by DeLean (DeLean, et al., Molecular Pharmacology, **21**, 5-16 (1982)). $IC_{50}$ values were converted to Ki values using the Cheng-Prusoff equation (Cheng, et al., Biochem. Pharmacol., **22**, 3099-3108 (1973)).

Representative compounds of the present invention were found to have affinity for the 5-$HT_{2c}$ receptor as measured essentially by the procedure described supra.

The 5-$HT_{2C}$ receptor is functionally coupled to specific G-proteins. Agonist activation of 5-$HT_{2C}$ G-protein-coupled receptors results in the release of GDP from the •-subunit (G alpha q or G alpha i) of the G-protein and the subsequent binding of GTP. The binding of the stable analog $[^{35}S]$-GTPγS is an indicator of this receptor's activation.

$[^{35}S]$-GTPγS binding

The immunoadsorption scintillation proximity assay (ISPA) in microtiter plates of $[^{35}S]$-GTPγS binding to G alpha q or G alpha i was modified from published conditions (DeLapp et al, JPET **289** (1999) 946-955). Test compounds were dissolved in DMSO and diluted in assay buffer consisting of 50 mM Tris-HCl (pH 7.4), 10 mM $MgCl_2$, 100 mM NaCl, and 0.2 mM EGTA. Incubations were performed over 12 test concentrations; volume was 200 μl. The incubation also contained 0.1 μM GDP and 0.25 nM $[^{35}S]$-GTPγS. Membrane homogenates from AV12 cells stably transfected with the human 5-$HT_{2C}$ receptor were added and the microtiter plates were incubated for 30 minutes at room temperature. The incubation was terminated by the addition of Nonidet P-40 (final. concentration of 0.27%), followed by addition of rabbit polyclonal anti-G alpha q/11 antibody (0.2 μg per well), and anti-rabbit scintillation proximity assay beads (Amersham; 1.25 mg per well; final volume was 290 μl). The mixture was incubated for 3 hours at room temperature to complete the immunoadsorption of $[^{35}S]$-GTPγS bound to G alpha q/11. Microtiter plates were centrifuged briefly to pellet beads. $[^{35}S]$-GTPγS binding was quantitated by microtiter plate scintillation spectrometry (Wallac). Data analysis was performed by nonlinear regression analysis with GraphPad Prism software running on a personal computer, using 5-HT control concentration-response curves to define maximal stimulation of $[^{35}S]$-GTPγS binding.

Representative compounds of the present invention were tested in the $[^{35}S]$-GTPγS assay and were found to be agonists of the 5-$HT_{2c}$ receptor.

The ability of agonists of the 5-$HT_{2c}$ receptor in general, and the compounds of the present invention in particular, to treat obesity is demonstrated by testing in a feeding assay.

Fasted Feeding Assay

Male rats were fasted for 18 hours prior to testing. Rats were first assigned to either a treatment or control group (N=8), then weighed, administered drug or vehicle orally, and returned to their home cage. Thirty minutes later, food was made available to the animals. The the food and the food hopper was weighed before, one hour, two hours,

and four hours after food was made available to the test animals. Weight of food consumed plus spillage by the treatment animals was compared to food consumed plus spillage by control animals using a one-way ANOVA, with a Dunnett's post-hoc test.

Representative compounds of the present invention were tested in the feeding assay and were found to reduce food consumed by fasting rats.

While it is possible to administer a compound employed in the methods of this invention directly without any formulation, the compounds are usually administered in the form of pharmaceutical compositions comprising a pharmaceutically acceptable excipient and at least one active ingredient. These compositions can be administered by a variety of routes including oral, rectal, transdermal, subcutaneous, intravenous, intramuscular, and intranasal. Many of the compounds employed in the methods of this invention are effective as both injectable and oral compositions. Such compositions are prepared in a manner well known in the pharmaceutical art and comprise at least one active compound. See, e.g. , REMINGTON'S PHARMACEUTICAL SCIENCES, (16th ed. 1980).

In making the compositions employed in the present invention the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches,. gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing from about 0.05 to about 100 mg, more usually about 1.0 to about 30 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical excipient.

The active compounds are generally effective over a wide dosage range. For examples, dosages per day normally fall within the range of about 0.01 to about 30 mg/kg. In the treatment of adult humans, the range of about 0.1 to about 15 mg/kg/day, in single or divided dose, is especially preferred. However, it will be understood that the amount of the compound actually administered will be determined by a physician, in the light of the relevant circumstances, including the condition to be treated, the chosen route of administration, the actual compound or compounds administered, the age, weight, and response of the individual patient, and the severity of the patient's symptoms, and therefore the above dosage ranges are not intended to limit the scope of the invention in any way. In some instances dosage levels below the lower limit of the aforesaid range may be more than adequate, while in other cases still larger doses may be employed without causing any harmful side effect, provided that such larger doses are first divided into several smaller doses for administration throughout the day.

Formulation Example 1

Hard gelatin capsules containing the following ingredients are prepared:

| Ingredient | Quantity (mg/capsule) |
|---|---|
| Compound of Example 1 | 30.0 |
| Starch | 305.0 |
| Magnesium stearate | 5.0 |

The above ingredients are mixed and filled into hard gelatin capsules in 340 mg quantities.

Formulation Example 2

A tablet formula is prepared using the ingredients below:

| Ingredient | Quantity (mg/tablet) |
|---|---|
| Compound of Example 2 | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

Formulation Example 3

A dry powder inhaler formulation is prepared containing the following components:

| Ingredient | Weight % |
|---|---|
| Compound of Example 3 | 5 |
| Lactose | 95 |

The active mixture is mixed with the lactose and the mixture is added to a dry powder inhaling appliance.

Formulation Example 4

Tablets, each containing 30 mg of active ingredient, are prepared as follows:

| Ingredient | Quantity (mg/tablet) |
|---|---|
| Compound of Example 4 | 30.0 mg |
| Starch | 45.0 mg |
| Microcrystalline cellulose | 35.0 mg |
| Polyvinylpyrrolidone (as 10% solution in water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U. S. sieve. The granules so produced are dried at 50-60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 120 mg.

Formulation Example 5

Capsules, each containing 40 mg of medicament are made as follows:

| Ingredient | Quantity (mg/capsule) |
|---|---|
| Compound of Example 5 | 40.0 mg |
| Starch | 109.0 mg |
| Magnesium stearate | 1.0 mg |
| Total | 150.0 mg |

The active ingredient, cellulose, starch, and magnesium stearate are blended, passed through a No. 20 mesh

U.S. sieve, and filled into hard gelatin capsules in 150 mg quantities.

Formulation Example 6

Suppositories, each containing 25 mg of active ingredient are made as follows:

| Ingredient | Amount |
|---|---|
| Compound of Example 6 | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

Formulation Example 7

Suspensions, each containing 50 mg of medicament per 5.0 ml dose are made as follows:

| Ingredient | Amount |
|---|---|
| Compound of Example 7 | 50.0 mg |
| Xanthan gum | 4.0 mg |
| Sodium carboxymethyl cellulose (11%) Microcrystalline cellulose (89%) | 50.0 mg |
| Sucrose | 1.75 g |
| Sodium benzoate | 10.0 mg |
| Flavor and Color | q.v. |
| Purified water to | 5.0 ml |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

Formulation Example 8

Capsules, each containing 15 mg of medicament, are made as follows:

| Ingredient | Quantity (mg/capsule) |
|---|---|
| Compound of Example 8 | 15.0 mg |
| Starch | 407.0 mg |
| Magnesium stearate | 3.0 mg |
| Total | 425.0 mg |

The active ingredient, cellulose, starch, and magnesium stearate are blended, passed through a No. 20 mesh U.S. sieve, and filled into hard gelatin capsules in 425 mg quantities.

Formulation Example 9

An intravenous formulation may be prepared as follows:

| Ingredient | Quantity |
|---|---|
| Compound of Example 9 | 250.0 mg |
| Isotonic saline | 1000 ml |

Formulation Example 10

A topical formulation may be prepared as follows:

| Ingredient | Quantity |
|---|---|
| Compound of Example 1 | 1-10 g |
| Emulsifying Wax | 30 g |
| Liquid Paraffin | 20 g |
| White Soft Paraffin | to 100 g |

The white soft paraffin is heated until molten. The liquid paraffin and emulsifying wax are incorporated and stirred until dissolved. The active ingredient is added and stirring is continued until dispersed. The mixture is then cooled until solid.

Formulation Example 11

Sublingual or buccal tablets, each containing 10 mg of active ingredient, may be prepared as follows:

| Ingredient | Quantity Per Tablet |
|---|---|
| Compound of Example 4 | 10.0 mg |
| Glycerol | 210.5 mg |
| Water | 143.0 mg |
| Sodium Citrate | 4.5 mg |
| Polyvinyl Alcohol | 26.5 mg |
| Polyvinylpyrrolidone | 15.5 mg |
| Total | 410.0 mg |

The glycerol, water, sodium citrate, polyvinyl alcohol, and polyvinylpyrrolidone are admixed together by continuous stirring and maintaining the temperature at about 90°C. When the polymers have gone into solution, the solution is cooled to about 50-55°C and the medicament is slowly admixed. The homogenous mixture is poured into forms made of an inert material to produce a drug-containing diffusion matrix having a thickness of about 2-4 mm. This diffusion matrix is then cut to form individual tablets having the appropriate size.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. See, e.g., U.S. Patent 5,023,252, issued June 11, 1991, herein incorporated by reference. Such patches may be constructed for continuous, pulsatile, or on demand delivery of pharmaceutical agents.

Frequently, it will be desirable or necessary to introduce the pharmaceutical composition to the brain, either directly or indirectly. Direct techniques usually involve placement of a drug delivery catheter into the host's ventricular system to bypass the blood-brain barrier. One such implantable delivery system, used for the transport of biological factors to specific anatomical regions of the body, is described in U.S. Patent 5,011,472, issued April 30, 1991, which is herein incorporated by reference.

Indirect techniques, which are generally preferred, usually involve formulating the compositions to provide for drug latentiation.by the conversion of hydrophilic drugs into lipid-soluble drugs or prodrugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

The type of formulation employed for the administration of the compounds employed in the methods of the present invention may be dictated by the particular compounds employed, the type of pharmacokinetic profile desired from the route of administration and the compound(s), and the state of the patient.

## Claims

1. The compounds of Formula I:

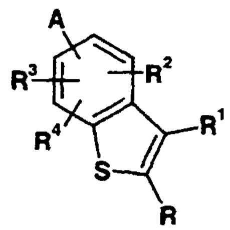

I

where:

R is hydrogen, halo, trifluoromethyl or $C_1$-$C_6$ alkyl;
$R^1$ is hydrogen, halo, trifluoromethyl, phenyl, or $C_1$-$C_6$ alkyl;
$R^2$, $R^3$, and $R^4$ are independently hydrogen, halo, trifluoromethyl, cyano, $C_1$-$C_4$ alkoxy, $C_1$-$C_4$ alkoxycarbonyl, $C_1$-$C_6$ alkyl, $C_1$-$C_6$ alkyl substituted with a substituent selected from the group consisting of $C_1$-$C_4$ alkoxy and hydroxy, or -C(O)NHR$^9$;
$R^9$ is $C_1$-$C_8$ alkyl where the alkyl chain is optionally substituted with a substituent selected from the group consisting of phenyl and pyridyl;
A is attached at either the 4- or 7-position of the benzofuran nucleus and is an amine of formula:

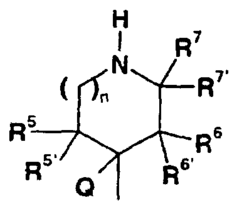

(i)

n is 0, 1, or 2;
$R^5$, $R^6$, and $R^7$ are independently hydrogen or $C_1$-$C_4$ alkyl;
Q is hydrogen;
$R^{5'}$ is hydrogen or methyl, provided that $R^{5'}$ may be methyl only when $R^5$ is other than hydrogen, or $R^{5'}$ and Q taken together with the carbon atoms to which they are attached form a double bond;
$R^{6'}$ is hydrogen or methyl, provided that $R^{6'}$ may be methyl only when $R^6$ is other than hydrogen, or $R^{6'}$ and Q taken together with the carbon atoms to which they are attached form a double bond;
$R^{7'}$ is hydrogen or methyl, provided that $R^{7'}$ may be methyl only when $R^7$ is other than hydrogen;

or pharmaceutically acceptable acid addition salts thereof subject to the following provisos:

a) when n is 1 or 2, at least one of $R^5$, $R^6$, and $R^7$, must be other than hydrogen; and
b) no more than two of $R^5$, $R^{5'}$, $R^6$, $R^{6'}$, $R^7$, and $R^{7'}$ may be other than hydrogen.

2. A compound of Claim 1 where A is attached at the 7-position of the benzofuran nucleus.

3. A compound according to either one of Claims 1 or 2 where Q is hydrogen.

4. A compound according to Claim 3 where $R^6$ is $C_1$-$C_4$ alkyl and $R^5$, $R^{5'}$, $R^7$ and $R^{7'}$ are each hydrogen.

5. A compound according to Claim 4 where $R^{6'}$ is hydrogen, and $R^6$ and the benzofuran core are in the cis configuration with regard to each other.

6. A compound according to either of Claim 4 or Claim 5 where $R^6$ is methyl.

7. A compound according to Claim 4 where $R^{6'}$ is methyl.

8. A compound according to either of Claim 1 or Claim 2 where one of $R^{5'}$ and Q, or $R^{6'}$ and Q, taken together with the carbon atoms to which they are attached form a double bond.

9. A compound according to Claim 8 where of $R^{5'}$ and Q, taken together with the carbon atoms to which they are attached form a double bond, $R^6$ is $C_1$-$C_4$ alkyl, and $R^{6'}$ is methyl.

10. A compound according to Claim 9 which is 3,3-dimethyl-4-(6-fluorobenzothien-7-yl)-1,2,3,6-tetrahydropyridine.

11. A pharmaceutical formulation which comprises, in association with a pharmaceutically acceptable carrier, diluent or excipient, a compound according to any one of Claims 1-10.

12. A compound according to any one of Claims 1-10 when used for increasing activation of the 5-$HT_{2C}$ receptor in mammals.

13. A compound according to any one of Claims 1-10 when used for the treatment of obesity in mammals.

14. A compound according to any one of Claims 1-10 when used for the treatment of depression in mammals.

15. A compound according to any one of Claims 1-10 when used for the treatment of obsessive compulsive disorder in mammals.

16. A compound according to any of Claims 12-15 where the mammal is human.

17. The use of a compound according to any one of Claims 1-10 in the manufacture of a medicament for increasing activation of the 5-$HT_{2C}$ receptor in mammals.

18. The use of a compound according to any one of Claims 1-10 in the manufacture of a medicament for the treatment of obesity in mammals.

19. The use of a compound according to any one of Claims 1-10 in the manufacture of a medicament for the treatment of depression in mammals.

20. The use of a compound according to any one of Claims 1-10 in the manufacture of a medicament for the treatment of obsessive compulsive disorder in mammals.

21. A use according to any of Claims 17-20 where the mammal is human.

**Patentansprüche**

1. Verbindungen der Formel I

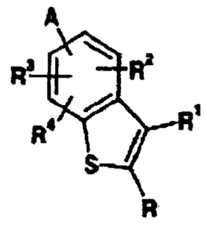

I

worin
R für Wasserstoff, Halogen, Trifluormethyl oder $C_1$-$C_6$-Alkyl steht,
$R^1$ für Wasserstoff, Halogen, Trifluormethyl, Phenyl oder $C_1$-$C_6$-Alkyl steht,
$R^2$, $R^3$ und $R^4$ unabhängig stehen für Wasserstoff. Halogen, Trifluormethyl, Cyano, $C_1$-$C_4$-Alkoxy, $C_1$-$C_4$-Alkoxycarbonyl, $C_1$-$C_6$-Alkyl, $C_1$-$C_6$-Alkyl, das substituiert ist mit einem Substituenten, der ausgewählt ist aus der Gruppe, die besteht aus $C_1$-$C_4$-Alkoxy und Hydroxy, oder für -C(O)NHR$^9$,
$R^9$ für $C_1$-$C_8$-Alkyl steht, worin die Alkylkette optional substituiert ist durch einen Substituenten, der ausgewählt ist aus der Gruppe, die besteht aus Phenyl und Pyridyl,
A entweder an die Position 4 oder die Position 7 des Benzofurankerns gebunden ist und für ein Amin der folgenden Formel steht

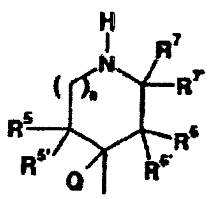

(i)

n für 0, 1 oder 2 steht,
$R^5$, $R^6$ und $R^7$ unabhängig für Wasserstoff oder $C_1$-$C_4$-Alkyl stehen,
Q für Wasserstoff sieht,
$R^{5'}$ für Wasserstoff oder Methyl steht, mit der Maßgabe, dass $R^{5'}$ nur dann Methyl sein kann, wenn $R^5$ etwas anderes als Wasserstoff ist, oder $R^{5'}$ und Q zusammen mit dem Kohlenstoffatom, an das sie gebunden sind, eine Doppelbindung biiden,
$R^{6'}$ für Wasserstoff oder Methyl steht, mit der Maßgabe, dass $R^{6'}$ nur dann Methyl sein kann wenn $R^6$ etwas anderes als Wasserstoff ist, oder $R^{6'}$ und Q zusammen mit dem Kohlenstoffatom, an das sie gebunden sind, eine Doppelbindung bilden,
$R^{7'}$ für Wasserstoff oder Methyl steht, mit der Maßgabe, dass $R^{7'}$ nur dann Methyl sein kann, wenn $R^7$ etwas anderes als Wasserstoff ist,
oder pharmazeutisch annehmbare Säureadditionssalze hiervon,
mit den Maßgaben, dass

a) falls n für 1 oder 2 steht, wenigstens einer der Substituenten $R^5$, $R^6$ und $R^7$ etwas anderes als Wasserstoff sein muss, und
b) nicht mehr als zwei der Substituenten $R^5$, $R^{5'}$, $R^6$, $R^{6'}$, $R^7$ und $R^{7'}$ etwas anderes als Wasserstoff sein können.

**2.** Verbindung nach Anspruch 1, worin A an die Position 7 des Benzofurankerns gebunden ist.

**3.** Verbindung nach Anspruch 1 oder 2, worin Q für Wasserstoff steht.

**4.** Verbindung nach Anspruch 3, worin $R^6$ für $C_1$-$C_4$-Alkyl steht und $R^5$, $R^{5'}$, $R^7$ und $R^{7'}$ jeweils Wasserstoff sind.

5. Verbindung nach Anspruch 4, worin $R^{6'}$ für Wasserstoff steht und $R^6$ und der Benzofurankern zueinander in cis-Konfiguration vorliegen.

6. Verbindung nach Anspruch 4 oder 5, worin $R^6$ für Methyl steht.

7. Verbindung nach Anspruch 4, worin $R^{6'}$ für Methyl steht.

8. Verbindung nach Anspruch 1 oder 2, worin einer von $R^{5'}$ und Q oder $R^{6'}$ und Q zusammen mit den Kohlenstoffatomen, an die sie gebunden sind, eine Doppelbindung bilden.

9. Verbindung nach Anspruch 8, worin $R^{5'}$ und Q zusammen mit den Kohlenstoffatomen, an die sie gebunden sind, eine Doppelbindung bilden, $R^6$ für $C_1$-$C_4$-Alkyl steht und $R^{6'}$ für Methyl steht.

10. Verbindung nach Anspruch 9, nämlich 3,3-Dimethyl-4-(6-fluorbezothien-7-yl)-1,2,3,6-tetrahydropyridin.

11. Pharmazeutische Formulierung, die in Assoziation mit einem pharmazeutisch annehmbaren Träger, Verdünnungsmittel oder Hilfsstoff eine Verbindung nach einem der Ansprüche 1 bis 10 umfasst.

12. Verbindung nach einem der Ansprüche 1 bis 10 zur Verwendung für eine Erhöhung der Aktivierung des 5-$HT_{2c}$-Rezeptors bei Säugern.

13. Verbindung nach einem der Ansprüche 1 bis 10 zur Verwendung für die Behandlung von Obesität bei Säugern.

14. Verbindung nach einem der Ansprüche 1 bis 10 zur Verwendung für die Behandlung von Depression bei Säugern.

15. Verbindung nach einem der Ansprüche 1 bis 10 zur Verwendung für die Behandlung von obsessiven kompulsiven Störungen bei Säugern.

16. Verbindung nach einem der Ansprüche 12 bis 15, worin der Säuger ein Mensch ist.

17. Verwendung der Verbindung nach einem der Ansprüche 1 bis 10 bei der Herstellung eines Arzneimittels zur Erhöhung einer Aktivierung des 5-$HT_{2c}$-Rezeptors bei Säugern.

18. Verwendung einer Verbindung nach einem der Ansprüche 1 bis 10 bei der Herstellung eines Arzneimittels zur Behandlung von Obesität bei Säugern.

19. Verwendung einer Verbindung nach einem der Ansprüche 1 bis 10 bei der Herstellung eines Arzneimittels zur Behandlung von Depression bei Säugern.

20. Verwendung einer Verbindung nach einem der Ansprüche 1 bis 10 bei der Herstellung eines Arzneimittels zur Behandlung obsessiver kompulsiver Störungen bei Säugern.

21. Verwendung nach einem der Ansprüche 17 bis 20, worin der Säuger ein Mensch ist.

## Revendications

1. Composés de formule I :

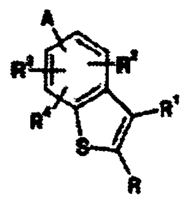

I

dans laquelle :

R représente un atome d'hydrogène, un atome d'halogène, un groupe trifluorométhyle ou un groupe alkyle en ($C_1$-$C_6$) ;
$R^1$ représente un atome d'hydrogène, un atome d'halogène, un groupe trifluorométhyle, un groupe phényle ou un groupe alkyle en ($C_1$-$C_6$) ;
$R^2$, $R^3$, et $R^4$ représentent indépendamment un atome d'hydrogène, un atome d'halogène, un groupe trifluorométhyle, un groupe cyano, un groupe alcoxy en ($C_1$-$C_4$), un groupe alcoxy en ($C_1$-$C_4$)carbonyle, un groupe alkyle en ($C_1$-$C_6$), un groupe alkyle en ($C_1$-$C_6$) substitué par un substituant choisi dans le groupe comprenant un groupe alcoxy en ($C_1$-$C_4$) et un groupe hydroxy, ou un groupe -C(O)NHR$^9$ ;
$R^9$ représente un groupe alkyle en ($C_1$-$C_8$) dans lequel la chaîne alkyle est éventuellement substituée par un substituant choisi dans le groupe comprenant un groupe phényle et un groupe pyridyle ;
A est attaché soit en position 4 soit en position 7 du noyau benzofurane et représente une amine de formule :

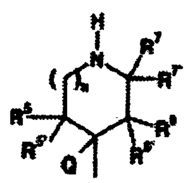

(i)

n vaut 0, 1, ou 2 ;
$R^5$, $R^6$, et $R^7$ représentent indépendamment un atome d'hydrogène ou un groupe alkyle en ($C_1$-$C_4$) ;
Q représente un atome d'hydrogène ;
$R^{5'}$ représente un atome d'hydrogène ou un groupe méthyle, pourvu que $R^{5'}$ puisse être un groupe méthyle uniquement lorsque $R^5$ est autre qu'un atome d'hydrogène, ou $R^{5'}$ et Q, pris conjointement avec les atomes de carbone auxquels ils sont attachés, forment une double liaison ;
$R^{6'}$ représente un atome d'hydrogène ou un groupe méthyle, pourvu que $R^{6'}$ puisse être un groupe méthyle uniquement lorsque $R^6$ est autre qu'un atome d'hydrogène, ou $R^{6'}$ et Q, pris conjointement avec les atomes de carbone auxquels ils sont attachés, forment une double liaison ;
$R^{7'}$ représente un atome d'hydrogène ou un groupe méthyle, pourvu que $R^{7'}$ puisse être un groupe méthyle uniquement lorsque $R^7$ est autre qu'un atome d'hydrogène ;

ou leurs sels d'addition d'acide pharmaceutiquement acceptables, soumis aux conditions suivantes :

a) lorsque n vaut 1 ou 2, au moins l'un de $R^5$, $R^6$, et $R^7$, doit être autre qu'un atome d'hydrogène ; et
b) pas plus de deux parmi $R^5$, $R^{5'}$, $R^6$, $R^{6'}$, $R^7$, et $R^{7'}$ peuvent être autres qu'un atome d'hydrogène.

**2.** Composé selon la revendication 1, dans lequel A est attaché en position 7 du noyau benzofurane.

**3.** Composé selon l'une des revendications 1 ou 2, dans lequel Q représente un atome d'hydrogène.

**4.** Composé selon la revendication 3, dans lequel $R^6$ représente un groupe alkyle en ($C_1$-$C_4$) et $R^5$, $R^{5'}$, $R^7$ et $R^{7'}$

représentent chacun un atome d'hydrogène.

**5.** Composé selon la revendication 4, dans lequel $R^{6'}$ représente un atome d'hydrogène, et $R^6$ et le noyau benzofurane sont dans la configuration cis l'un par rapport à l'autre.

**6.** Composé selon l'une des revendications 4 et 5, dans lequel $R^6$ représente un groupe méthyle.

**7.** Composé selon la revendication 4, dans lequel $R^{6'}$ représente un groupe méthyle.

**8.** Composé selon l'une des revendications 1 ou 2, dans lequel l'un de $R^{5'}$ et Q, ou de $R^{6'}$ et Q, pris conjointement avec les atomes de carbone auxquels ils sont attachés, forment une double liaison.

**9.** Composé selon la revendication 8, dans lequel $R^{5'}$ et Q, pris conjointement avec les atomes de carbone auxquels ils sont attachés, forment une double liaison, $R^6$ représente un groupe alkyle en ($C_1$-$C_4$), et $R^{6'}$ représente un groupe méthyle.

**10.** Composé selon la revendication 9, qui est la 3,3-diméthyl-4-(6-fluorobenzothién-7-yl)-1,2,3,6-tétrahydropyridine.

**11.** Formulation pharmaceutique qui comprend, en association avec un support, un diluant ou un excipient pharmaceutiquement acceptable, un composé selon l'une quelconque des revendications 1 à 10.

**12.** Composé selon l'une quelconque des revendications 1 à 10 lorsqu'il est utilisé pour accroître l'activation du récepteur 5-$HT_{2C}$ chez les mammifères.

**13.** Composé selon l'une quelconque des revendications 1 à 10 lorsqu'il est utilisé pour le traitement de l'obésité chez les mammifères.

**14.** Composé selon l'une quelconque des revendications 1 à 10 lorsqu'il est utilisé pour le traitement de la dépression chez les mammifères.

**15.** Composé selon l'une quelconque des revendications 1 à 10 lorsqu'il est utilisé pour le traitement d'un trouble obsessionnel compulsif chez les mammifères.

**16.** Composé selon l'une quelconque des revendications 12 à 15 où le mammifère est un humain.

**17.** Utilisation d'un composé selon l'une quelconque des revendications 1 à 10 pour la fabrication d'un médicament destiné à accroître l'activation du récepteur 5-$HT_{2C}$ chez les mammifères.

**18.** Utilisation d'un composé selon l'une quelconque des revendications 1 à 10 pour la fabrication d'un médicament destiné au traitement de l'obésité chez les mammifères.

**19.** Utilisation d'un composé selon l'une quelconque des revendications 1 à 10 pour la fabrication d'un médicament destiné au traitement de la dépression chez les mammifères.

**20.** Utilisation d'un composé selon l'une quelconque des revendications 1 à 10 pour la fabrication d'un médicament destiné au traitement d'un trouble obsessionnel compulsif chez les mammifères.

**21.** Utilisation selon l'une quelconque des revendications 17 à 20 où le mammifère est un humain.